(19)

**Europäisches Patentamt**
**European Patent Office**
**Office européen des brevets**

(11) **EP 4 039 825 A1**

(12) **EUROPEAN PATENT APPLICATION**

(43) Date of publication:
**10.08.2022 Bulletin 2022/32**

(21) Application number: **21155943.0**

(22) Date of filing: **09.02.2021**

(51) International Patent Classification (IPC):
*C12Q 1/6881* (2018.01)    *C12Q 1/6883* (2018.01)
*C12Q 1/6886* (2018.01)

(52) Cooperative Patent Classification (CPC):
**C12Q 1/6881; C12Q 1/6883; C12Q 1/6886;**
C12Q 2600/158; C12Q 2600/16

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BOUWMAN, Wilbert Hendrik**
**5656 AE Eindhoven (NL)**
• **VERHAEGH, Wilhelmus Franciscus Johannes**
**5656 AE Eindhoven (NL)**
• **VAN DE STOLPE, Anja**
**5656 AE Eindhoven (NL)**

(74) Representative: **Philips Intellectual Property & Standards**
**High Tech Campus 52**
**5656 AG Eindhoven (NL)**

(54) **COMPARISON AND STANDARDIZATION OF CELL AND TISSUE CULTURE**

(57) The invention relates to a method for characterizing a cell or tissue culture sample based on multiple cellular signaling pathway activities. The cellular signaling pathway activities are preferably determined based on expression levels of target genes of the respective pathways. The invention further describes products useful for the method having primers for the detection of pathway target genes, and uses of the product.

| Sample | Number of replicates | ER Score ± SD | AR Score ± SD | PI3K Score ± SD |
|---|---|---|---|---|
| MDA-MB-231 cells 18 hrs doxycycline induced FOXO | 5 | 8.4 ± 3.3 | 17.7 ± 0.9 | 31.8 ± 3.9 |
| MDA-MB-231 cells untreated | 5 | 7.0 ± 1.1 | 17.2 ± 0.7 | 73.7 ± 0.9 |

| Sample | MAPK Score ± SD | HH Score ± SD | NOTCH Score ± SD | TGFβ Score ± SD |
|---|---|---|---|---|
| MDA-MB-231 cells 18 hrs doxycycline induced FOXO | 65.2 ± 1.1 | 26.6 ± 2.5 | 14.6 ± 1.3 | 22.2 ± 1.4 |
| MDA-MB-231 cells untreated | 61.6 ± 1.4 | 31.4 ± 2.3 | 16.7 ± 1.2 | 20.3 ± 1.4 |

Fig. 1

EP 4 039 825 A1

**Description**

FIELD OF THE INVENTION

The present invention is in the field of life sciences. In particular, the invention relates to cell and tissue culture, and to methods for characterizing a cell line or tissue culture based on a sample thereof, or performing quality control on a cell line or tissue culture.

BACKGROUND OF THE INVENTION

Coordinated activity of a relatively small number of signal transduction pathways control embryonic development, and therefore the cell division, migration and differentiation of stem cells with varying differentiation potential. The signaling pathways are evolutionary well conserved and control these basic cellular mechanisms [1],[2],[3],[4]. Signaling pathways can be roughly categorized as hormonal driven nuclear receptor pathways (e.g. androgen and estrogen receptor pathways), developmental pathways (e.g. Wnt, Hedgehog, TGFβ, Notch pathways), the inflammatory NFκB pathway, and the highly complex growth factor regulated signaling pathway network, in which the PI3K-AKT-mTOR pathway is probably the most prominent one, next to MAPK and JAK-STAT pathways [2],[4]. In addition to embryonic development they control important physiological processes, e.g. hematopoiesis and generation of an immune response, tissue regeneration, hair growth, and renewal of intestinal mucosa.

In physiological cellular processes their activity is tightly controlled. However, they also play important roles in the pathophysiology of many, if not all, diseases, for example benign and malignant tumors, auto-immune (e.g. rheumatoid arthritis) and immunodeficiency diseases, and neurological diseases (e.g. epilepsy). In diseases, control of their activity is typically altered or lost [5],[6],[7],[8],[9],[10],[11],[12],[13],[14],[15]. In cancer this is exemplified by uncontrolled cell division compared to a strictly controlled balance between cell division and differentiation in the developing embryo, while distant metastasis can be seen as an uncontrolled version of cell migration during organ development [16],[17]. It is thought that cancer cells which have acquired certain stem cell features are the main cells which metastasize.

An increasing number of drugs is being developed with the aim to target components of these signaling pathways to modulate their activity in a growing number of diseases, e.g. cancer, auto-immune diseases [18],[19],[20]. Their use in many other diseases is being pioneered in clinical studies. Development of these drugs requires in depth knowledge on the role of signaling pathways in disease pathophysiology. Optimal clinical use of targeted drugs requires assessment of signaling pathway activity on an individual patient basis. For such purposes in vitro cell and tissue culture-based disease models are well suited, on the premise that the cultured model is sufficiently representative for the diseases.

Use of cell lines in biomedical and pharma research however poses a reproducibility problem. Cell lines form the backbone of biomedical research and drug development. However, even when genetically identical, they may differ with respect to phenotype (RNA and/or protein expression). This presents a huge problem for experimental reproducibility and may provide an explanation for the large percentage of non-reproducible biomedical publications (60-70%). This also causes problems in drug development, since response to drugs varies with cellular phenotype [21].

Therefore, there is a need to test or characterize cells in culture to enable more standardized testing conditions. These problems, among others, are overcome by the invention as outlined in the appended claims.

SUMMARY OF THE INVENTION.

The present invention is based on the realization of the inventors that quantitative readouts of cellular signaling pathways can be used to identify and compare samples. Recent developments of the inventors and others has led to the development of quantitative methods to accurately and consistently determine the activity of cellular signaling pathways in a sample. Currently such methods have been described for the ER, AR, PI3K/FOXO, MAPK-AP1, HH, NOTCH, TGFbeta, NFkB, STAT1/2, STAT3 and WNT pathways, among others. More interestingly, all these pathway activities can be determined simultaneously in a sample without the need for expensive equipment or reagents. This allows the generation of a pathway "fingerprint" consisting of the numerical values representing a multitude of cellular signaling pathway activities for a sample, which can further be compared to for example a reference fingerprint.

Therefore, in a first embodiment the invention relates to a method for testing a cell line based on a sample obtained from said cell line, wherein the method is based on a cellular signaling pathway fingerprint, wherein the fingerprint is defined as a plurality of numerical values representing distinct cellular signaling pathway activities in a sample, and wherein the testing comprises comparing the cellular signaling pathway fingerprint to a reference cellular signaling pathway fingerprint.

In an embodiment the testing further comprises:

- determining the expression levels of at least three target genes for each cellular signaling pathway in the fingerprint.

In an embodiment the expression levels are based on mRNA isolated from the sample.

In an embodiment the testing further comprises isolating mRNA from the sample.

In an embodiment the fingerprint comprises four or more cellular signaling pathways selected from the ER, AR, PI3K/FOXO, MAPK-AP1, HH, NOTCH, TGFbeta, NFkB, STAT1/2, STAT3 and WNT pathways.

In an embodiment the three or more genes of the ER pathway are selected from the group consisting of CDH26, SGK3, PGR, GREB1, CA12, XBP1, CELSR2, WISP2, DSCAM, ERBB2, CTSD, TFF1 and NRIP1, optionally further comprising one or more target genes selected from AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;

the three or more genes of the AR pathway are selected from the group consisting of KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally further comprising one or more target genes selected from APP, NTS, PLAU, CDKN1A, DRG1, FGF8, IGF1, PRKACB, PTPN1, SGK1 and TACC2;

the three or more genes of the PI3K/FOXO pathway are selected from the group consisting of AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10, optionally further comprising one or more target genes selected from ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4, optionally further comprising one or more target genes selected from ATG14, BIRC5, IGFBP1, KLF2, KLF4, MYOD1, PDK4, RAG1, RAG2, SESN1, SIRT1, STK11 and TXNIP;

the three or more genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;

the three or more genes of the HH pathway are selected from the group consisting of GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally further comprising one or more target genes selected from BCL2, FOXA2, FOXF1, H19, HHIP, IL1R2, JAG2, JUP, MIF, MYLK, NKX2.2, NKX2.8, PITRM1 and TOM1;

the three or more genes of the NOTCH pathway are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;

the three or more genes of the TGFbeta pathway are selected from the group consisting of ANGPTL4, CDC42EP3, CDKN1A, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;

the three or more genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;

the three or more genes of the STAT1/2 pathway are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1, and USP18, or the three or more genes of the STAT1/2 pathway are selected from the group consisting of GBP1, IRF9, STAT1, TAP1, ISG15, APOL1, IRF1, IRF7, IFI6, IFITM1, USP18, CXCL9, OAS1, APOL2, IFIT2 and LY6E;

the three or more genes of the STAT3 pathway are selected from the group consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;

the three or more genes of the WNT pathway are selected from the group consisting of KIAA1199, AXIN2, RNF43, TBX3, TDGF1, SOX9, ASCL2, IL8, SP5, ZNRF3, KLF6, CCND1, DEFA6 and FZD7, optionally further comprising one or more target genes selected from NKD1, OAT, FAT1, LEF1, GLUL, REG1B, TCF7L2, COL18A1, BMP7, SLC1A2, ADRA2C, PPARG, DKK1, HNF1A and LECT2.

In an embodiment, the testing is used to:

- determine the passage number of the cell line; or
- determine whether the cell line is within an acceptable range of passages; or
- to confirm or identify the identity of a cell line; or
- to determine the effect of a condition on a cell line; or
- to perform a quality control on the cell line; or
- to compare different samples; or
- to select for a suitable cell model system to mimic a disease.

In a second aspect, the invention relates to a product for determining the cellular signaling pathway activities of four or more cellular signaling pathways selected from the ER, AR, PI3K/FOXO, MAPK-AP1, HH, NOTCH, TGFbeta, NFkB, STAT1/2, STAT3 and WNT pathways, wherein the pathway activity is based on the expression levels of three or more target genes for each cellular signaling pathway, wherein the product comprises primers and optionally probes for determining the expression levels of said three or more target genes for each cellular signaling pathway, and wherein the three or more genes of the ER pathway are selected from the group consisting of CDH26, SGK3, PGR, GREB1, CA12, XBP1, CELSR2, WISP2, DSCAM, ERBB2, CTSD, TFF1 and NRIP1, optionally further comprising one or more target genes selected from AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;
the three or more genes of the AR pathway are selected from the group consisting of KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally further comprising one or more target genes selected from APP, NTS, PLAU, CDKN1A, DRG1, FGF8, IGF1, PRKACB, PTPN1, SGK1 and TACC2;
the three or more genes of the PI3K/FOXO pathway are selected from the group consisting of AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10, optionally further comprising one or more target genes selected from ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4, optionally further comprising one or more target genes selected from ATG14, BIRC5, IGFBP1, KLF2, KLF4, MYOD1, PDK4, RAG1, RAG2, SESN1, SIRT1, STK11 and TXNIP;
the three or more genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;
the three or more genes of the HH pathway are selected from the group consisting of GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally further comprising one or more target genes selected from BCL2, FOXA2, FOXF1, H19, HHIP, IL1R2, JAG2, JUP, MIF, MYLK, NKX2.2, NKX2.8, PITRM1 and TOM1;
the three or more genes of the NOTCH pathway are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more genes of the TGFbeta pathway are selected from the group consisting of ANGPTL4, CDC42EP3, CDKN1A, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;
the three or more genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;

the three or more genes of the STAT1/2 pathway are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1, and USP18, or the three or more genes of the STAT1/2 pathway are selected from the group consisting of GBP1, IRF9, STAT1, TAP1, ISG15, APOL1, IRF1, IRF7, IFI6, IFITM1, USP18, CXCL9, OAS1, APOL2, IFIT2 and LY6E;

the three or more genes of the STAT3 pathway are selected from the group consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;

the three or more genes of the WNT pathway are selected from the group consisting of KIAA1199, AXIN2, RNF43, TBX3, TDGF1, SOX9, ASCL2, IL8, SP5, ZNRF3, KLF6, CCND1, DEFA6 and FZD7, optionally further comprising one or more target genes selected from NKD1, OAT, FAT1, LEF1, GLUL, REG1B, TCF7L2, COL18A1, BMP7, SLC1A2, ADRA2C, PPARG, DKK1, HNF1A and LECT2.

In a third aspect the invention relates to use of the product according to the second aspect in the method according to first aspect of the invention.

In an embodiment, the use is for:

- determining the passage number of the cell line; or
- determining whether the cell line is within an acceptable range of passages; or
- confirming or identifying the identity of a cell line; or
- determining the effect of a condition on a cell line; or
- performing a quality control on the cell line; or
- comparing different samples; or
- to select for a suitable cell model system to mimic a disease.

DETAILED DESCRIPTION OF THE INVENTION

It was found by the inventors that pathway activities may be a beneficial tool in assessing several properties of a sample, such as a cell or tissue culture sample. Due to recent developments of the inventors and others, it is now possible to determine quantitatively in a short time frame pathway activities of multiple cellular signaling pathways in a sample, based on expression levels of target genes of the respective pathways. For example by using qPCR on the extracted mRNA of the sample, the expression levels of a number of genes may be determined, and these expression levels can then be used to calculate a numerical value representing the pathway activity associated with the target genes. It has already been demonstrated that multiple pathways may thus be analyzed in a single amplification reaction, by using target genes of multiple pathways in a single reaction, for example on a 96 well plate. In principle, by increasing the number of wells used, or by using multiplexing, the number of pathways that can be analyzed simultaneously is nearly limitless, allowing for a quick way to determine pathway activity of many pathways in a single sample simultaneously.

The inventors found that the different pathway activities are relatively constant in sample which have been treated under the same conditions. It was further found that certain pathway activities increase or decrease in a predictable manner under certain conditions. This has led to the realization that a "fingerprint" can be made of a sample by analyzing multiple pathway activities in that sample, and that the fingerprint can hold useful information about the sample. For example, the fingerprint can be used as a quality control tool as described below.

Therefore, in a first embodiment the invention relates to a method for testing a cell line based on a sample obtained from said cell line, wherein the method is based on a cellular signaling pathway fingerprint, wherein the fingerprint is defined as a plurality of numerical values representing distinct cellular signaling pathway activities in a sample, and wherein the testing comprises comparing the cellular signaling pathway fingerprint to a reference cellular signaling pathway fingerprint. Preferably the cellular signaling pathway activities are each determined based on the expression levels of target genes, preferably three or more target genes.

The reference cellular signaling pathway fingerprint may be obtained independently. The reference cellular signaling pathway fingerprint is defined as a plurality of numerical values representing distinct cellular signaling pathway activities in a reference sample. Preferably the cellular signaling pathway activities comprised in the reference cellular signaling pathway fingerprint are each determined based on the expression levels of target genes, preferably three or more target genes. Preferably, the expression levels are based on mRNA isolated from the reference sample. Preferably the reference cellular signaling pathway fingerprint comprises four or more, for example four, five, six, seven, eight, nine, ten or eleven, cellular signaling pathways selected from the ER, AR, PI3K/FOXO, MAPK-AP1, HH, NOTCH, TGFbeta, NFkB, STAT1/2, STAT3 and WNT pathways. Particularly preferred is that the cellular signaling pathways comprised in the cellular signaling pathway fingerprint overlap at least partially with the cellular signaling pathways comprised in the reference cellular signaling pathway fingerprint. More preferably all the cellular signaling pathways comprised in the cellular signaling pathway fingerprint are also comprised in the reference cellular signaling pathway fingerprint.

In an embodiment the testing further comprises:

- determining the expression levels of at least three, e.g. three, four, five, six, seven, eight, nine, ten, eleven, twelve or more, target genes for each cellular signaling pathway in the fingerprint.

In an embodiment the expression levels are based on mRNA isolated from the sample.

In an embodiment the testing further comprises isolating mRNA from the sample.

In an embodiment the fingerprint comprises four or more, for example four, five, six, seven, eight, nine, ten or eleven, cellular signaling pathways selected from the ER, AR, PI3K/FOXO, MAPK-AP1, HH, NOTCH, TGFbeta, NFkB, STAT1/2, STAT3 and WNT pathways. It is understood that increasing the number of cellular signaling pathways used in the fingerprint will increase the amount of information comprised in the fingerprint, and by extend its application, therefore preferably more than four cellular signaling pathways are used.

*1. Sample collection and RNA isolation*

When used herein, a cell line refers to any type of cell or tissue culture. Non limiting examples of a cell line when used herein are: immortalized cells, primary cells, tissue cultures, organoids, 3D cell cultures, cell suspension cultures. The cell line may refer to human derived cells, animal derived cell, plant derived cells or micro-organism derived cells, such as bacterial, prokaryote, fungal, or yeast cells. The cell line may also refer to a tissue culture, such as a cultured biopsy, or part of an organ. The terms cell line, cell culture and tissue culture are used interchangeably herein.

When used herein, sample refers to a sample obtained from the cell line as defined herein. The sample may refer to the living cells, fixed cells, lysed cells or cells having undergone any type of treatment, e.g. to isolate the mRNA or DNA. Typically the sample at least contains or comprises the mRNA and/or the DNA of the cells from the cell culture it refers to. For example, the sample may refer to whole cells scraped from a culture plate or dish. Sample may also refer to lysed cells. For example, for RNA extraction using trizol,

Methods for isolating mRNA from a sample are known to the skilled person and have for example been described in Molecular Cloning: A Laboratory Manual (Fourth Edition) - A laboratory manual by M.R. Green and J. Sambrook, Cold Spring Harbor Laboratory Press. A non-limiting example protocol for isolating mRNA suitable to be used in the methods described here is:
Homogenization:

1. Tissues: Homogenize tissue samples in 1 ml of TRIZOL reagent per 50 to 100 mg of tissue using a glass-Teflon or power homogenizer. The sample volume should not exceed 10% of the volume of TRIZOL Reagent used for the homogenization.
2. Cells grown in Monolayer: Rinse cell monolayer with ice cold PBS once. Lyse cells directly in a culture dish by adding 1 ml of TRIZOL Reagent per 3.5 cm diameter dish and scraping with cell scraper. Pass the cell lysate several times through a pipette. Vortex thoroughly. The amount of TRIZOL reagent added is based on the area of the culture dish (1 ml per 10 cm2) and not on the number of cells present. An insufficient amount of TRIZOL Reagent may result in DNA contamination of the isolated RNA.
3. Cells Grown in suspension: Spin cells for 5 min at 300 X g. Remove media and resuspend cells in ice cold PBS. Pellet cells by spinning at 300 X g for 5 min. Lyse cells with TRIZOL Reagent by repetitive pipetting or by passing through syringe and needle. Use 1 ml of the reagent per 5-10 X 10^6 of animal cells.
4. Incubate the homogenized sample for 5 minutes at room temperature to permit the complete dissociation of nucleoprotein complexes. Centrifuge to remove cell debris. Transfer the supernatant to new tube.

PHASE SEPERATION: Add 0.2 ml of chloroform per 1 ml of TRIZOL Reagent. Cap sample tubes securely. Vortex samples vigorously for 15 seconds and incubate them at room temperature for 2 to 3 minutes. Centrifuge the samples at no more than 12,000 x g for 15 minutes at 2 to 80°C. Following centrifugation, the mixture separates into lower red, phenolchloroform phase, an interphase, and a colorless upper aqueous phase. RNA remains exclusively in the aqueous phase. Transfer upper aqueous phase carefully without disturbing the interphase into fresh tube. Measure the volume of the aqueous phase (The volume of the aqueous phase is about 60% of the volume of TRIZOL Reagent used for homogenization).

RNA PRECIPITATION: Precipitate the RNA from the aqueous phase by mixing with isopropyl alcohol. Use 0.5 ml of isopropyl alcohol per 1 ml of TRIZOL Reagent used for the initial homogenization. Incubate samples at 15 to 30° C for 10 minutes and centrifuge at not more than 12,000 x g for 10 minutes at 2 to 4°C. The RNA precipitate, often invisible before centrifugation, forms a gel-like pellet on the side and bottom of the tube.

RNA WASH: Remove the supernatant completely. Wash the RNA pellet once with 75% ethanol, adding at least 1 ml of 75% ethanol per 1 ml of TRIZOL Reagent used for the initial homogenization. Mix the samples by vortexing and centrifuge at no more than 7,500 x g for 5 minutes at 2 to 8 °C. Repeat above washing procedure once. Remove all leftover ethanol.

REDISSOLVING RNA: Air-dry or vacuum dry RNA pellet for 5-10 minutes. Do not dry the RNA pellet by centrifuge under vacuum. It is important not to let the RNA pellet dry completely as this will greatly decrease its solubility. Partially dissolved RNA samples have an A260/A280 ratio < 1.6. Dissolve RNA in DEPC-treated water by passing solution a few times through a pipette tip.

The term sample may further refer to the product obtained in any intermediate step as obtained in above or in comparable protocols for mRNA isolation or extraction.

*2. Determining expression levels of target genes*

When used herein, "expression level" refers to quantifying the number of mRNA copies transcribed from a gene. Generally this number will not be an absolute value but a relative value, and therefore is preferably normalized for example in reference to the expression of one or more housekeeping genes. Housekeeping genes are genes which are assumed to have constant expression levels independent of cell type and/or functional status of the cell (i.e. from a diseased or healthy subject), and therefore can be used to normalize experimentally determined relative expression levels. Housekeeping genes are generally known to the skilled person, non-limiting examples of housekeeping genes that may be used for normalization are beta-actin, glyceraldehyde-3-phosphate dehydrogenase (GAPDH) and Transcription factor IID TATA binding protein (TBP).

Therefore the phrase "expression level of a target gene" denotes a value that is representative of the amount, e.g. a concentration, of a target gene present in a sample. This value may be based on the amount of a transcription product of the target gene (e.g. mRNA) or a translation product thereof (e.g. protein). Preferably, the expression level is based on the amount of mRNA formed from the target gene. In order to determine the expression level, techniques such as qPCR, multiple qPCR, multiplexed qPCR, ddPCR, RNAseq, RNA expression array or mass spectrometry may be used. For example, a gene expression microarray, e.g. Affymetrix microarray, or RNA sequencing methods, like an Illumina sequencer, can be used.

*3. Pathway analysis*

The method of the invention is based on the finding that a "fingerprint" can be made for a sample based on a plurality of cellular signaling pathway activities. The cellular signaling pathway activity should be determined in a quantitative manner, so the fingerprint essentially is a plurality of numerical values representing the corresponding cellular signaling pathway activities.

When used herein, the terms "fingerprint" and "pathway fingerprint" are used interchangeably and refer to the collective information represented by numeric values of multiple cellular signaling pathway activities. The fingerprint may for example comprise three, four, five, six, seven eight, nine or ten or more cellular signaling pathway activities. For example, when for a sample the Wnt, HH, NFkB and STAT3 have been determined, the fingerprint [$P_{WNT}$, $P_{HH}$, $P_{NFkB}$, $P_{STAT3}$] may look like [45, 35, 14, 20], wherein "P" represents the respective pathway activities and the activities are represented by a relative number, in this case a value ranging from 0 to 100. The fingerprint may hold pathway activity values from a single pathway activity measurement for each pathway, or the pathway values for each cellular signaling pathway may be the average of multiple measurements. The multiple measurements may be from the same sample (technical replicates) or different samples. The fingerprint may hold additional information, e.g. the standard deviation in case an average pathway value is used.

When used herein, the term "reference fingerprint" refers to the fingerprint of a sample with a known state. For example the known state may be the passage number or a specific treatment of the cell line. A reference fingerprint may be used as a comparison to determine whether the pathway activities are the same or are not the same. For this purpose the reference fingerprint is preferably based on multiple samples so that the signaling pathway activities are based on an average, and that e.g. standard deviations can be calculated. In such case a pathway activity can be determined the same if the pathway activity in the fingerprint and the reference fingerprint are for example within two, preferably one standard deviations. Alternatively the pathway activity can be determined to higher or lower (with respect to the pathway activity in the reference fingerprint) when the pathway activity is more than one, preferably two, standard deviation higher or lower than the pathway activity in the reference fingerprint.

Preferred methods for determining cellular signaling activity have been described in WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications WO2018096076 (titled "Method to distinguish tumor suppressive FOXO activity from oxidative stress"), WO2019068585 (titled "Assessment of Notch cellular signaling pathway activity using mathematical modelling of target gene expression"), WO2019120658 (titled "Assessment of MAPK-MAPK-AP1 cellular signaling pathway activity using mathematical modelling of target gene expression"), WO2019068543 (titled "Assessment of JAK-JAK-STAT3 cellular signaling pathway activity using mathematical modelling of target gene expression"), WO2019068562 (titled "Assessment of JAK-STAT1/2 cellular signaling pathway activity using mathematical modelling of target gene expression"), and WO2019068623 (titled "Determining functional status of immune cells types and immune response"); each of these is incorporated by reference in its entirety. The methods describe therein are summarized below.

It is noted however that other quantitative methods can be used in a similar way for the purpose of the claimed methods. For example a simple model can be constructed where the pathway activity is quantified as a simple addition of the normalized and quantified expression levels of the three or more target genes, optionally the normalized and quantified expression levels of the three or more target genes may individually be multiplied by a constant to correct for relative contribution or negative correlation of the respective target genes. For example the following formula could be used:

$$P = c(1) * tg(1) + c(2) * tg(2) + \cdots + c(n) * tg(n)$$

Where *tg(x)* represents the quantified and normalized expression level of target gene x, *c(x)* represents a constant for target gene x, and P represents the pathway activity. The constants for each target gene can be experimentally determined to correct for relative contribution of the target gene to the pathway activity, and can be negative in case a negative correlation exists between target gene expression and pathway activity (meaning increased pathway activity directly correlates with reduced expression levels of the respective target gen).

Now the fingerprint can for example be represented as the collective numeric values for each of the determined pathway activities, e.g. a pathway activity selected from ER, AR, P13K/FOXO, MAPK-AP1, HH, NOTCH, TGFbeta, NFkB, STAT1/2, STAT3 and WNT.

For improved accuracy of the pathway activity for each cellular signaling pathway can be determined as described in patent documents referenced above. The models described therein use either a linear or a Bayesian model to calculate a pathway activity based on the expression levels of three or more target genes.

The terms pathway, signaling pathway and cellular signaling pathway arte used herein interchangeably and refer to the signaling cascade resulting from the respective factor or receptor, typically resulting in up- or downregulation of specific genes regulated by said factor or receptor, referred herein as target genes. The factor may for example be a growth factor or a hormone. The receptor may be a transmembrane receptor or a receptor present in the cytosol or the nucleus.

The following abbreviations are used to refer to the different cellular signaling pathways: Estrogen receptor (ER), androgen receptor (AR), phosphatidylinositol 3-kinase / Forkhead box O (PI3K/FOXO), Mitogen-activated protein kinase - Activator protein 1 (MAPK-AP1), Hedgehog (HH), NOTCH, Transforming growth factor beta (TGFbeta), nuclear factor kappa-light-chain-enhancer of activated B cells (NFkB), signal transducer and activator of transcription 1/2 (STAT1/2), signal transducer and activator of transcription 3 (STAT3) and Wingless and Int-1 (WNT). These cellular signaling pathways are well known to the skilled person in the field.

Alternatively, pathway models as described before and referred above may be used to quantitatively determine pathway activity. The above referenced cellular pathway activity models have been biologically validated for ER, AR, PI3K-FOXO, HH, Notch, TGF-β, Wnt, NFkB, JAK-STAT1/2, JAK-STAT3 and MAPK-AP1 pathways on several cell types.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as a cell present in a sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the cell present in a sample based on the determined activity level of the signaling pathway associated TF element. As described herein, the activity level can be directly used as an input to determine the fingerprint, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., "Selection of personalized patient therapy through the use of knowledge-based computational models that identify tumor-driving signal transduction pathways", Cancer Research, Vol. 74, No. 11, 2014, pages 2936 to 2945.

Particularly preferred is a method wherein the inferring of the cellular signaling pathway is based on three or more, e.g. three, four, five, six, seven, eight, nine, ten, eleven, twelve or more, target genes, wherein:

the three or more, e.g. three, four, five, six, seven, eight, nine, ten, eleven, twelve or more, genes of the ER pathway are selected from the group consisting of CDH26, SGK3, PGR, GREB1, CA12, XBP1, CELSR2, WISP2, DSCAM, ERBB2, CTSD, TFF1 and NRIP1, optionally further comprising one or more, e.g. one, two, three, four, five, six, seven, eight, nine, ten, eleven, twelve or more, target genes selected from AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25; the three or more, e.g. three, four, five, six, seven, eight, nine, ten, eleven, twelve or more, genes of the AR pathway are selected from the group consisting of KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally further comprising one or more, e.g. one, two, three, four, five, six, seven, eight, nine, ten, eleven, twelve or more, target genes selected from APP, NTS, PLAU, CDKN1A, DRG1, FGF8, IGF1, PRKACB, PTPN1, SGK1 and TACC2;

the three or more, e.g. three, four, five, six, seven, eight, nine, ten, eleven, twelve or more, genes of the PI3K/FOXO pathway are selected from the group consisting of AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10, optionally further comprising one or more, e.g. one, two, three, four, five, six, seven, eight, nine, ten, eleven, twelve or more, target genes selected from ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4, optionally further comprising one or more, e.g. one, two, three, four, five, six, seven, eight, nine, ten, eleven, twelve or more, target genes selected from ATG14, BIRC5, IGFBP1, KLF2, KLF4, MYOD1, PDK4, RAG1, RAG2, SESN1, SIRT1, STK11 and TXNIP;

the three or more, e.g. three, four, five, six, seven, eight, nine, ten, eleven, twelve or more, genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;

the three or more, e.g. three, four, five, six, seven, eight, nine, ten, eleven, twelve or more, genes of the HH pathway are selected from the group consisting of GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally further comprising one or more, e.g. one, two, three, four, five, six, seven, eight, nine, ten, eleven, twelve or more, target genes selected from BCL2, FOXA2, FOXF1, H19, HHIP, IL1R2, JAG2, JUP, MIF, MYLK, NKX2.2, NKX2.8, PITRM1 and TOM1;

the three or more, e.g. three, four, five, six, seven, eight, nine, ten, eleven, twelve or more, genes of the NOTCH pathway are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, one, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;

the three or more, e.g. three, four, five, six, seven, eight, nine, ten, eleven, twelve or more, genes of the TGFbeta pathway are selected from the group consisting of ANGPTL4, CDC42EP3, CDKN1A, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;

the three or more, e.g. three, four, five, six, seven, eight, nine, ten, eleven, twelve or more, genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;

the three or more, e.g. three, four, five, six, seven, eight, nine, ten, eleven, twelve or more, genes of the STAT1/2 pathway are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1, and USP18, or the three, e.g. three, four, five, six, seven, eight, nine, ten, eleven, twelve or more, or more genes of the STAT1/2 pathway are selected from the group consisting of GBP1, IRF9, STAT1, TAP1, ISG15, APOL1, IRF1, IRF7, IFI6, IFITM1, USP18, CXCL9, OAS1, APOL2, IFIT2 and LY6E;

the three or more, e.g. three, four, five, six, seven, eight, nine, ten, eleven, twelve or more, genes of the STAT3 pathway are selected from the group consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;

the three or more, e.g. three, four, five, six, seven, eight, nine, ten, eleven, twelve or more, genes of the WNT pathway are selected from the group consisting of KIAA1199, AXIN2, RNF43, TBX3, TDGF1, SOX9, ASCL2, IL8, SP5, ZNRF3, KLF6, CCND1, DEFA6 and FZD7, optionally further comprising one or more, e.g. one, two, three, four, five, six, seven, eight, nine, ten, eleven, twelve or more, target genes selected from NKD1, OAT, FAT1, LEF1, GLUL, REG1B, TCF7L2, COL18A1, BMP7, SLC1A2, ADRA2C, PPARG, DKK1, HNF1A and LECT2.

It was previously demonstrated that by using the carefully selected and curated lists of target genes for each cellular signaling pathway described, a random selection of three target genes suffices to determine pathway activity in a quantitative, reliable and repeatable manner, and for example suffices to distinguish between different states based on pathway activity. It will be clear however to the skilled person that the pathway models will become increasingly more accurate by the inclusion of additional target genes. Therefore in a preferred embodiment more than three target genes are used.

Herein, an ER transcription factor (TF) element is defined to be a protein complex containing at least one or preferably a dimer of nuclear Estrogen receptor, which is capable of binding to specific DNA sequences, thereby controlling transcription of target genes. Preferably, the term refers to either a protein or protein complex transcriptional factor triggered by the binding of estrogen to its receptor or an intermediate downstream signaling agent between the binding of estrogen to its receptor and the final transcriptional factor protein or protein complex.

Herein, an AR transcription factor (TF) element is defined to be a protein complex containing at least one or preferably a dimer of nuclear Androgen receptor, which is capable of binding to specific DNA sequences, thereby controlling transcription of target genes. Preferably, the term refers to either a protein or protein complex transcriptional factor triggered by the binding of androgenic hormones such as testosterone to its receptor or an intermediate downstream signaling agent between the binding of androgenic hormones such as testosterone to its receptor and the final transcriptional factor protein or protein complex.

Herein, a FOXO transcription factor (TF) element is defined to be a protein complex containing at least one of the FOXO TF family members, i.e., FOXO1, FOXO3A, FOXO4 and FOXO6, which is capable of binding to specific DNA sequences, thereby controlling transcription of target genes, which is capable of binding to specific DNA sequences, thereby controlling transcription of target genes.

Herein, the term "AP-1 transcription factor element" or "AP-1 TF element" or "TF element" when referring to the MAPK-AP1 pathway is defined to be a protein complex containing at least a member of the Jun (e.g. c-Jun, JunB and JunD) family and/or a member of the Fos (e.g. c-Fos, FosB, Fra-1 and Fra-2) family and/or a member of the ATF family and/or a member of the JDP family, forming e.g. Jun~Jun or Jun~Fos dimers, capable of binding to specific DNA sequences, preferably the response elements 12-0-Tetradecanoylphorbol-13-acetate (TPA) response element (TRE) with binding motif 5'-TGA G/C TCA-3' or cyclic AMP response element (CRE) with binding motif 5'-TGACGTCA-3', thereby controlling transcription of target genes. Preferably, the term refers to either a protein or protein complex transcriptional factor triggered by the binding of AP-1 inducing ligands, such as growth factors (e.g., EGF) and cytokines, to its receptor or an intermediate downstream signaling agent, or triggered by the presence of an AP-1 -activating mutation.

Herein, the term "Hedgehog transcription factor element" or "HH TF element" or "TF element" when referring to the Hedgehog cellular signaling pathway refers to a nuclear complex comprising at least one protein of the Gli family, e.g. Gli1, Gli2 or Gli3, which is capable of binding to specific DNA sequences, thereby controlling transcription of target genes. Preferably, the term refers to either a protein or protein complex transcriptional factor triggered by the binding of HH to its receptor or an intermediate downstream signaling agent between the binding of HH to its receptor and the final transcriptional factor protein or protein complex.

Herein, the term "Notch transcription factor element" or "Notch TF element" or "TF element" is defined to be a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-Jκ, Su(H) and LAG-1), which is capable of binding to specific DNA sequences, and preferably one co-activator protein from the mastermind-like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

Herein, the term "TGFbeta transcription factor element" or "TGFbeta TF element" or "TF element" when referring to the TGFbeta pathway is defined to be a protein complex containing at least one or, preferably, a dimer of the TGFbeta members (SMAD1, SMAD2, SMAD3 , SMAD5 and SMAD8 with SMAD4) or a trimer (two proteins from SMAD1, SMAD2, SMAD3, SMAD5 and SMAD8 with SMAD4), which is capable of binding to specific DNA sequences, thereby controlling transcription of target genes. Preferably, the term refers to either a protein or protein complex transcriptional factor triggered by the binding of TGFbeta to its receptor or an intermediate downstream signaling agent between the binding of TGFbeta to its receptor and the final transcriptional factor protein or protein complex. For example, it is known that TGFbeta binds to an extracellular TGFbeta receptor that initiates an intracellular "SMAD" signaling pathway and that one or more SMAD proteins (receptor-regulated or R-SMADs (SMAD1, SMAD2, SMAD 3, SMAD5 and SMAD8) and SMAD4) participate in, and may form a hetero-complex which participates in, the TGFbeta transcription signaling cascade which controls expression.

Herein, an NFkB transcription factor (TF) element is defined to be a protein complex containing at least one or, preferably, a dimer of the NFkB members (NFKB 1 or p50/p105, NFKB2 or p52/p100, RELA or p65, REL, and RELB), which is capable of binding to specific DNA sequences, thereby controlling transcription of target genes.

Herein, the term "JAK-STAT1/2 transcription factor element" or "JAK- STAT1/2 TF element" or "TF element" when referring to the JAK-STAT1/2 is defined to be a protein complex containing at least a STAT1-STAT2 heterodimer or a STAT1 homodimer, which is capable of binding to specific DNA sequences, preferably the ISRE (binding motif AGTTTC NTTCNC/T) or GAS (binding motif TTC/A NNG/TAA) response elements, respectively, thereby controlling transcription of target genes. Preferably, the term refers to either a protein or protein complex transcriptional factor that is formed by different stimuli such as IFNs triggered by the binding of the stimulating ligand to its receptor resulting in downstream signaling.

Herein, the term "JAK-STAT3 transcription factor element" or "JAK-STAT3 TF element" or "TF element" when referring to the JAK-STAT3 pathway is defined to be a protein complex containing at least a STAT3 homodimer, which is capable of binding to specific DNA sequences, preferably the response elements with binding motif CTGGGAA, thereby controlling transcription of target genes. Preferably, the term refers to either a protein or protein complex transcriptional factor triggered by the binding of STAT3 inducing ligands such as interleukin-6 (IL-6) and IL-6 family cytokines to its receptor or an intermediate downstream signaling agent between the binding the ligand to its receptor and the final transcriptional factor protein or protein complex.

Herein, a Wnt transcription factor (TF) element is defined to be a protein complex containing at least one of the TCF/LEF TF family members, i.e., TCF1, TCF3, TCF4 or LEF1, preferably wherein the Wnt TF element comprises beta-catenin/TCF4, which is capable of binding to specific DNA sequences, thereby controlling transcription of target genes.

When used herein, relative expression level refers to the quantitative value obtained for the expression level of the gene which has been normalized, e.g. by using a housekeeping gene or genes, to correct for sample variation.

*4. Applications of the method*

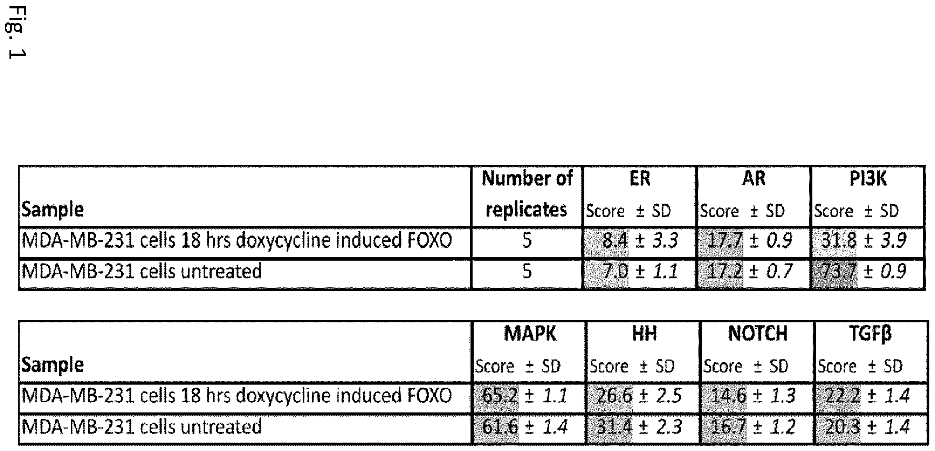

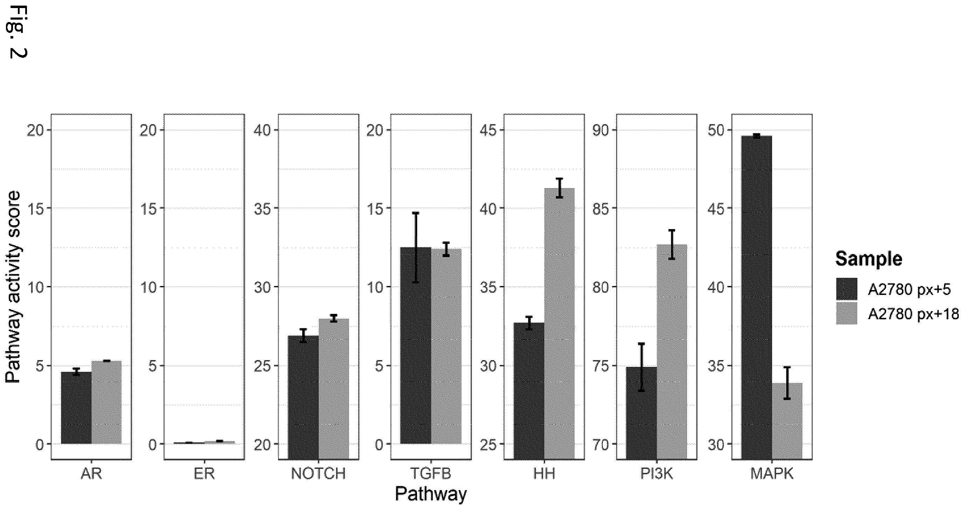

It was found by the inventors that cellular signaling pathway activities are very constant when compared among the same cell line when identical conditions are used, as for example demonstrated by Fig. 1, where in 5 replicates the standard deviation for most pathway scores is between 1 and 2, on a score scale from 0 to 100. Fig.1 further demonstrates that successful manipulation of cells can easily be followed using the pathway fingerprint. The relevance of this pathway fingerprint is demonstrated by Fig.2, where the same cells are cultured at identical conditions, but samples are taken from cells at passage 5 and passage 18. Even though the cells are derived from exactly the same line and cultured in identical conditions, it is apparent from the pathway fingerprints that activities of certain cellular signaling pathways is very different between the passages. This may have a huge impact on experiments conducted with these cells, therefore careful characterization of pathway activities is desirable to allow reproducibility of experimental results.

The use of the pathway fingerprint is envisioned, among others, in the following scenarios:

*A. Quality control*

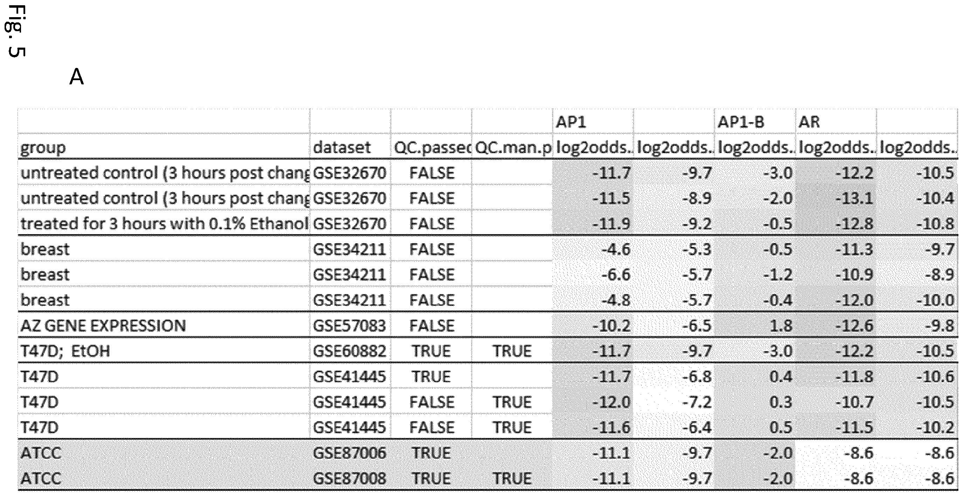

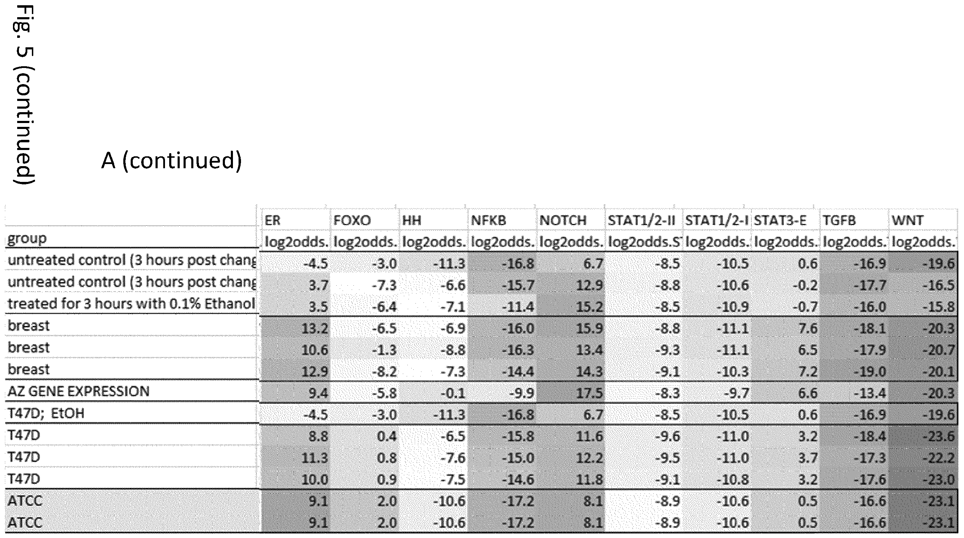

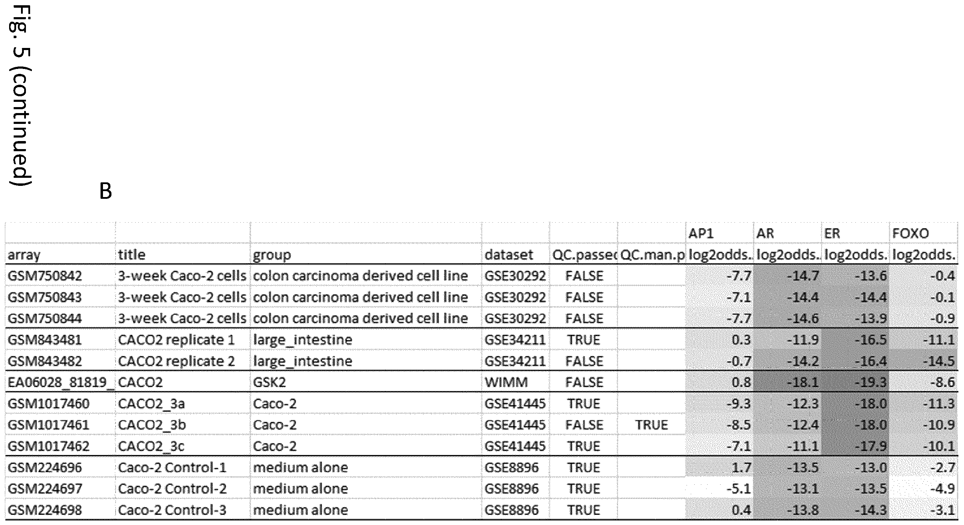

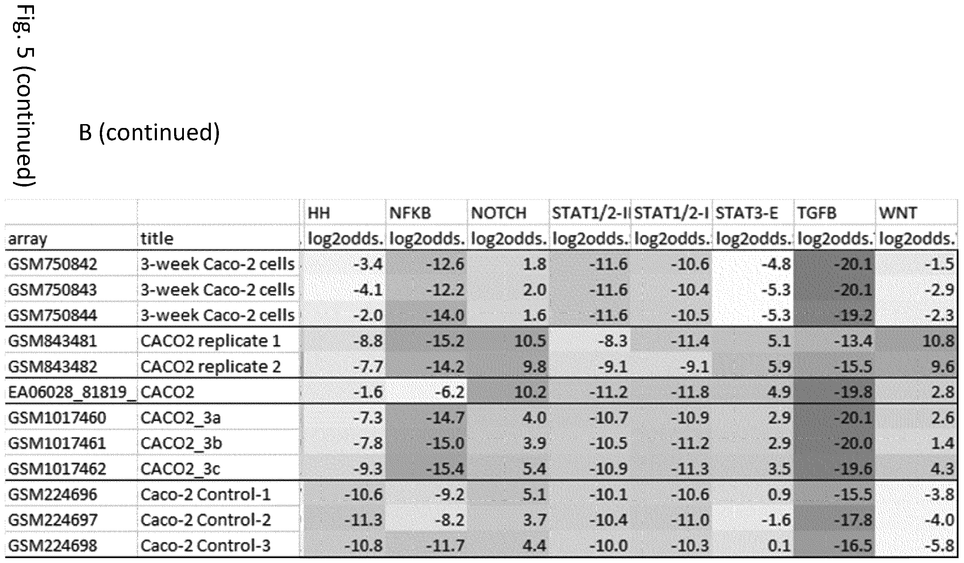

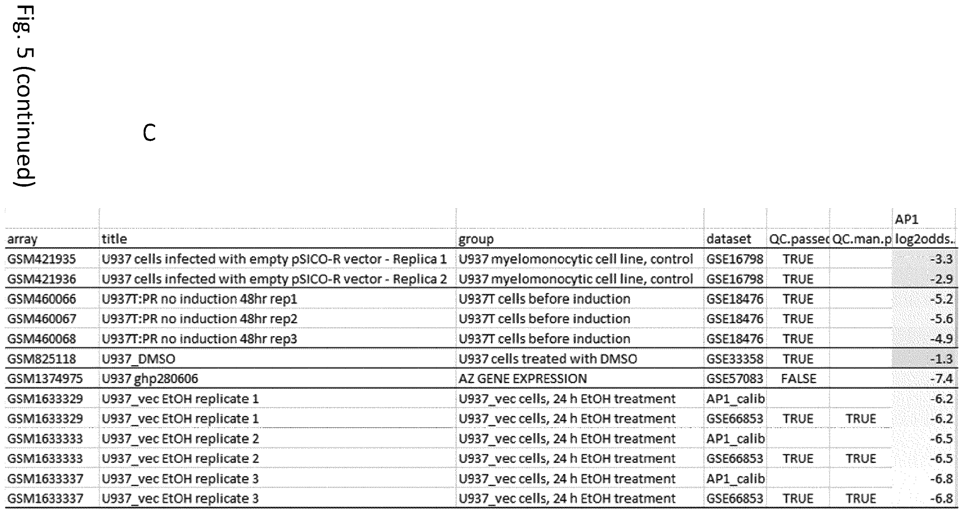

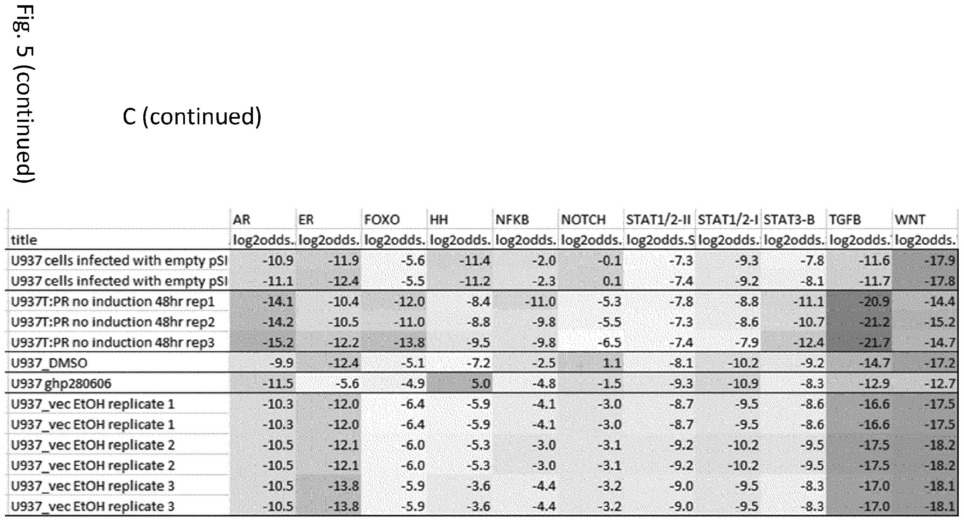

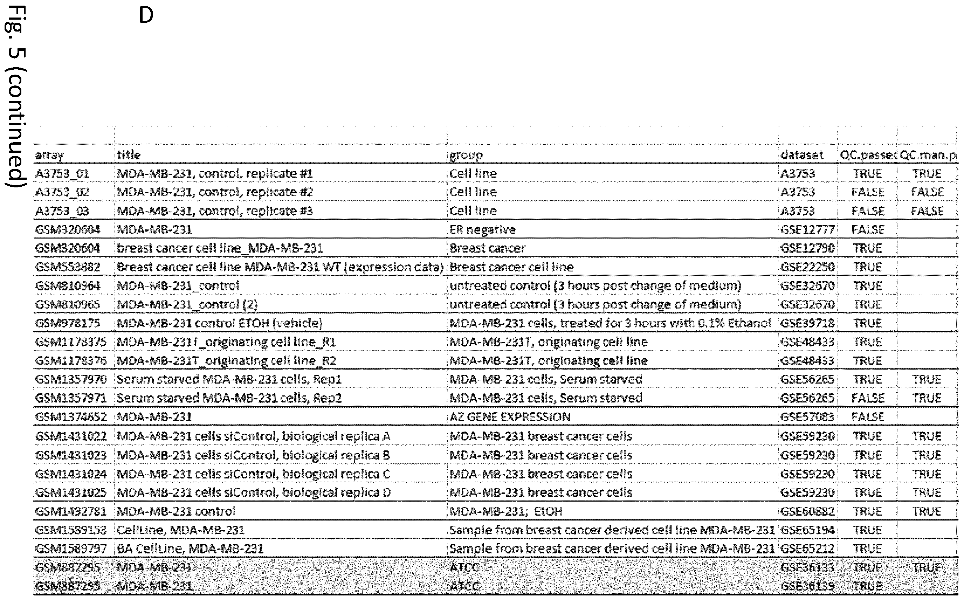

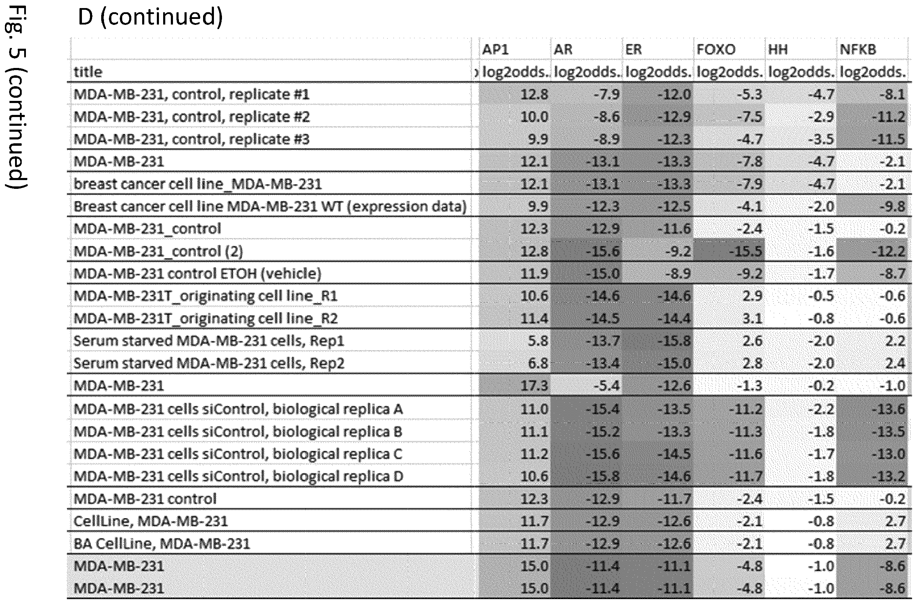

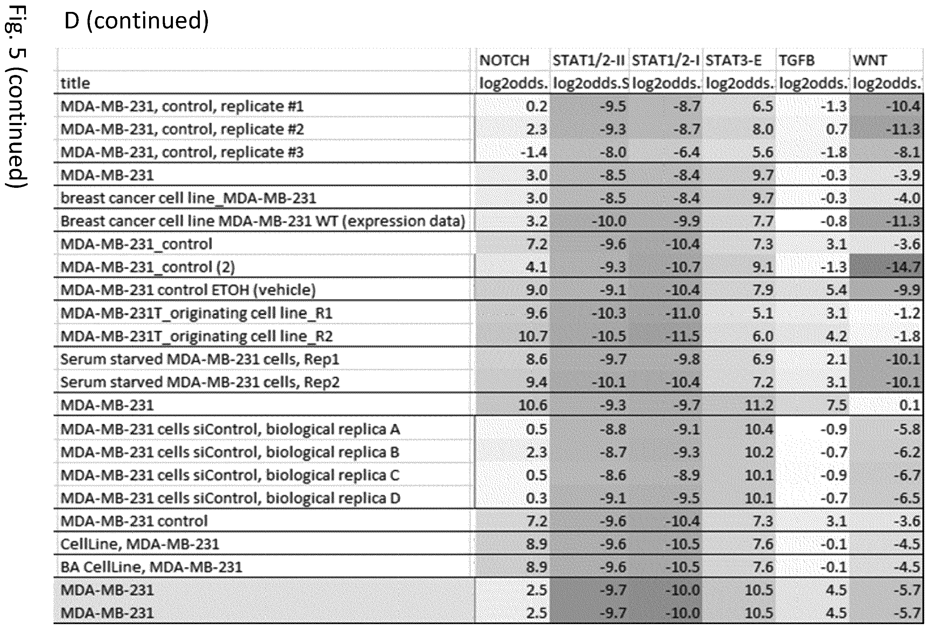

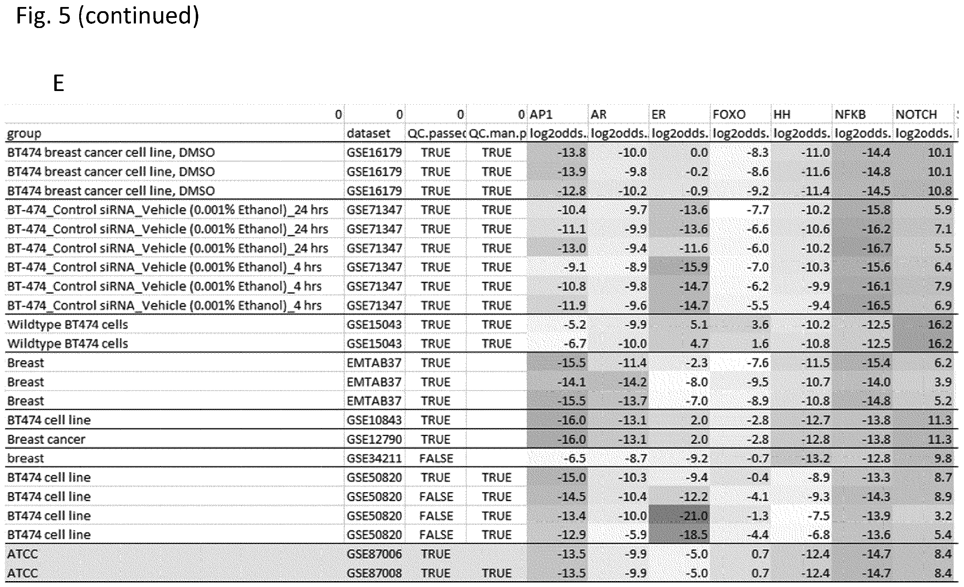

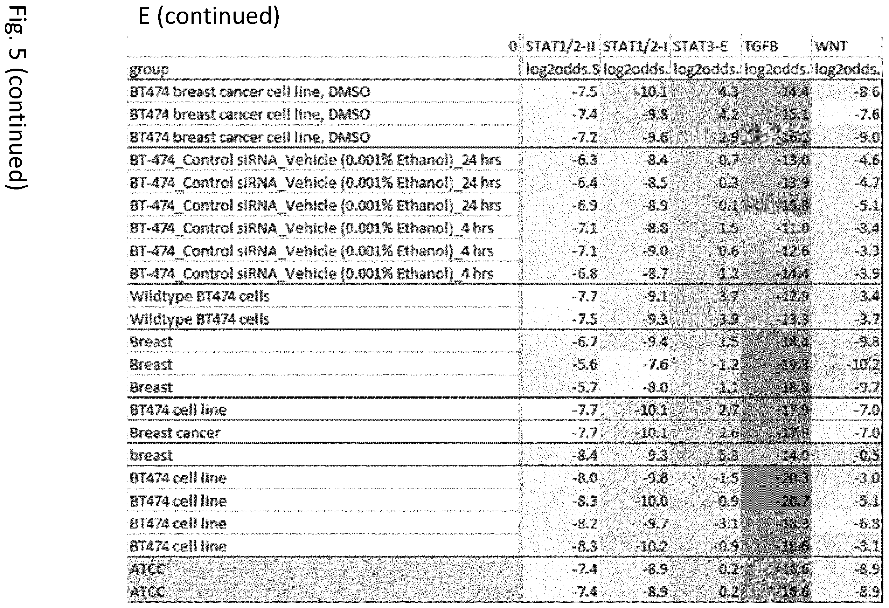

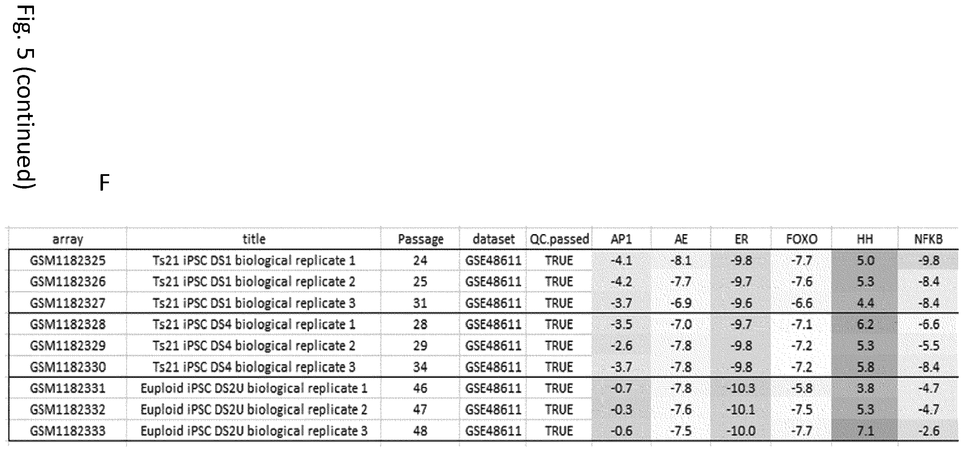

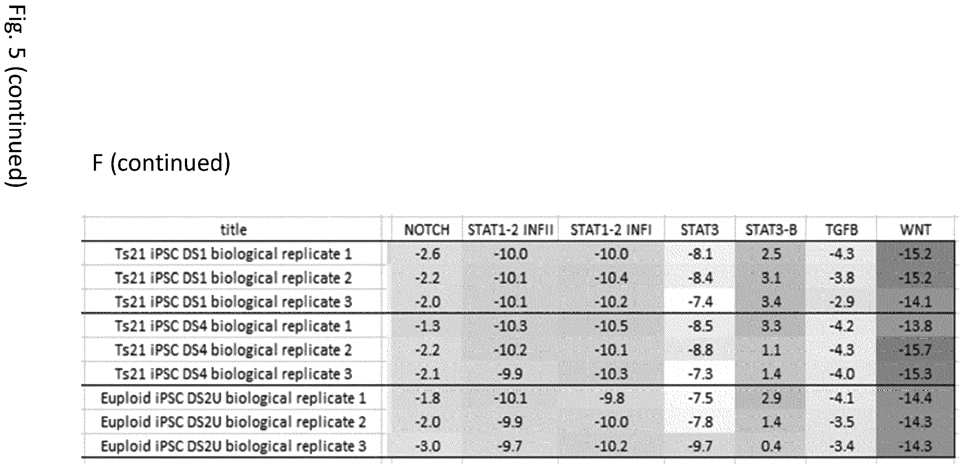

The pathway fingerprint may be used to serve as a quality control between experiments performed with different passage numbers of a cell line. By performing a pathway fingerprint on a subset of cells which are used for a relevant experiment, it can easily be determined in a later experiment (e.g. a repeat or follow up experiment) if the same fingerprint is obtained, thereby verifying the biological outcome of the experiment, or alternatively when it results in a different fingerprint explain possible deviations or unexpected results. Examples of this application are provided in the data represented by Fig. 5.

The phenomenon that cells kept in culture will "change over time" due to accrued mutations and selection of certain subpopulations of cells is exhaustively described in the literature. It is the reason that "immortalized" cell lines are kept until a certain passage number is reached before they are discarded. The passage number until when cell can be kept in culture is however not accurately determined for each cell line. Moreover this may vary due to other conditions, such as the amount of passages performed in a week, the culture medium used and other variables. Therefore, cells may be used for experiments when already displaying deviating behavior, or can be unnecessarily discarded. This can be easily avoided by the pathway fingerprint, as the fingerprint will provide by way of pathway activities as measured in the sample reveal whether the expected pathway activities are obtained or deviations are starting to show associated with higher passage numbers. It is further envisioned that by creating reference fingerprints for a cell line for multiple passage numbers, the passage number of a sample may be estimated by comparing its fingerprint with the multiple reference fingerprints and identifying its closest match. Examples of this application are provided in the data represented by Figs. 2, 3 and 6. Therefore, in an embodiment the fingerprint is used to determine if the cell line is within an acceptable passage number based on the fingerprint.

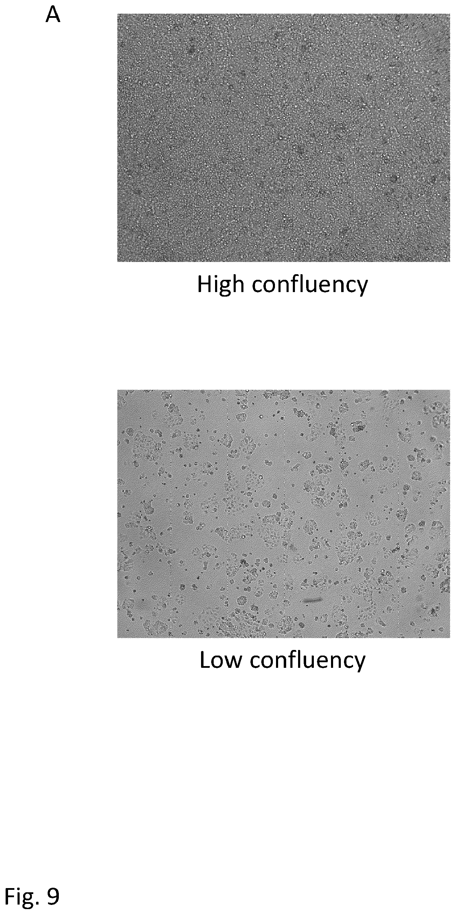

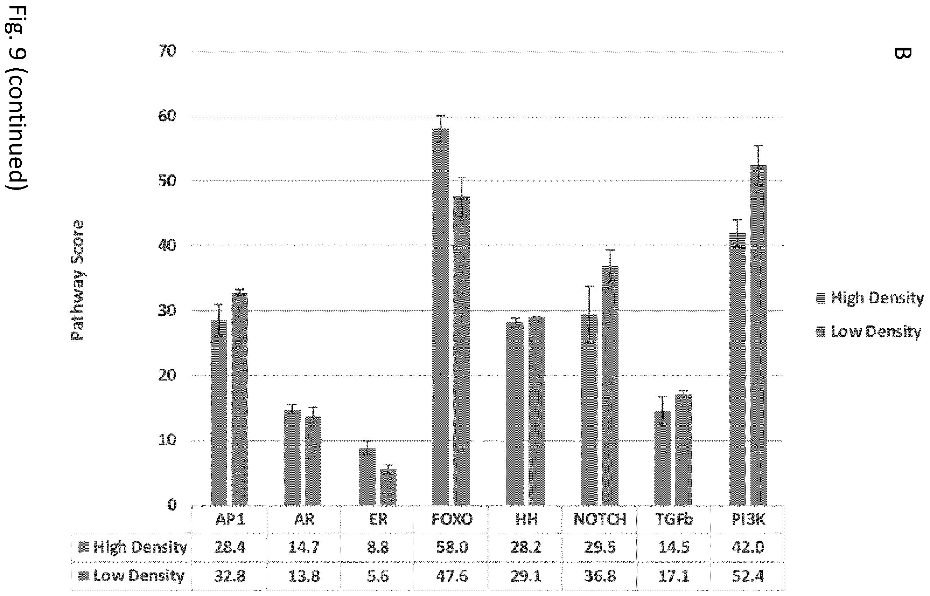

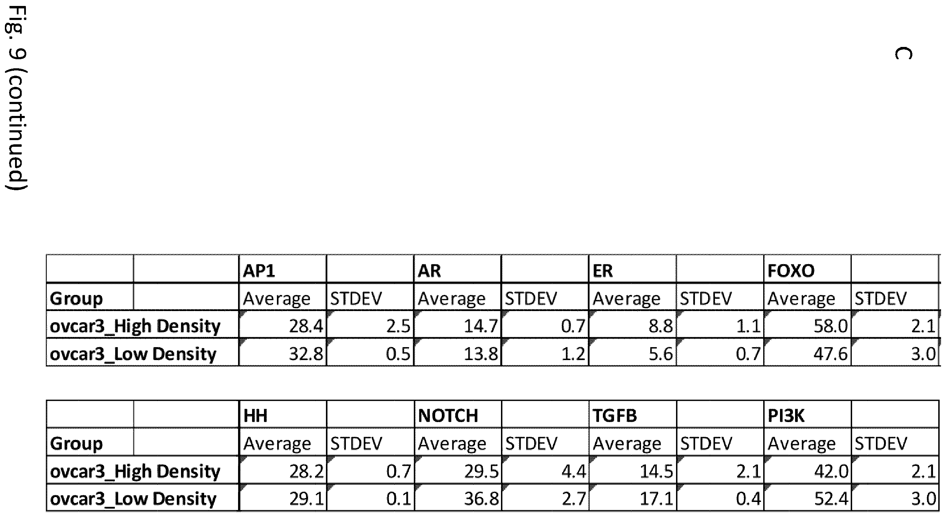

It is further found by the inventors that cell density has a strong influence on pathway activities and may therefore influence experimental results if low and high confluent cells are compared. Therefore, the fingerprint may be used to control for influences of different confluency on experimental design. Therefore, in an embodiment the fingerprint is used to determine or compare the confluency of the sample. Examples of this application are provided in the data represented by Fig. 9.

A different frequently encountered problem is that cell lines get contaminated with different cells, which may eventually out-populate the original cells, or cells simply get unknowingly swapped for different cells. Verifying that a cell line is indeed the correct cell line can also be done using pathway fingerprint, as different cell lines will display very specific pathway fingerprints. By obtaining the pathway fingerprint from a sample and comparing it to a reference fingerprint form the assumed cell line, it can easily be revealed if all the measured pathway activities are within the expected error margins or deviate considerably suggesting the cell line is either a different cell line as expected or is at least contaminated with a different cell line or behaving differently. It is further envisioned that by creating a library of fingerprint references for a multitude of cell lines, an unknown cell line may be identified by matching its fingerprint to a corresponding reference fingerprint of a known cell line.

Alternatively the method may be used to determine a cell source. For example a cell fingerprint for ATCC and a fingerprint for other cell culture bank for same cell line are generated and a fingerprint obtained from a sample can be compared to these fingerprint to determine its source.

*B. Experimental readout*

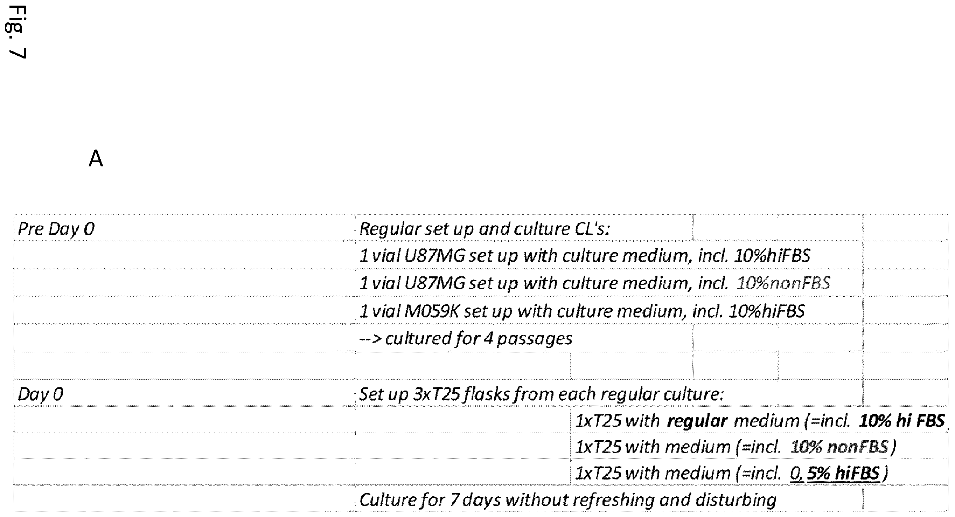

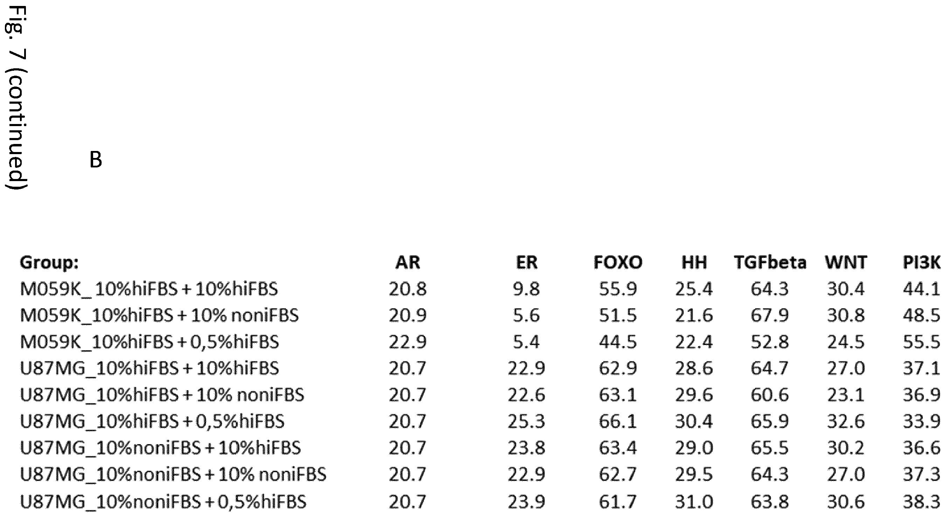

The pathway fingerprint can further be used in experimental readout, e.g. when a pathway inhibitor is used on a cell line it can be used to verify that indeed the pathway activity is reduced. The advantage of using a fingerprint for this purpose, instead of only looking at the respective signaling pathway, is that the fingerprint will easily show if other pathways remain unaffected or have also changed activity. This may for example arise from unexpected cross-talk between signaling pathway, or due to non-specific cross-reactivity of the compound being tested. Examples of this application are provided in the data represented by Figs. 1 and 7.

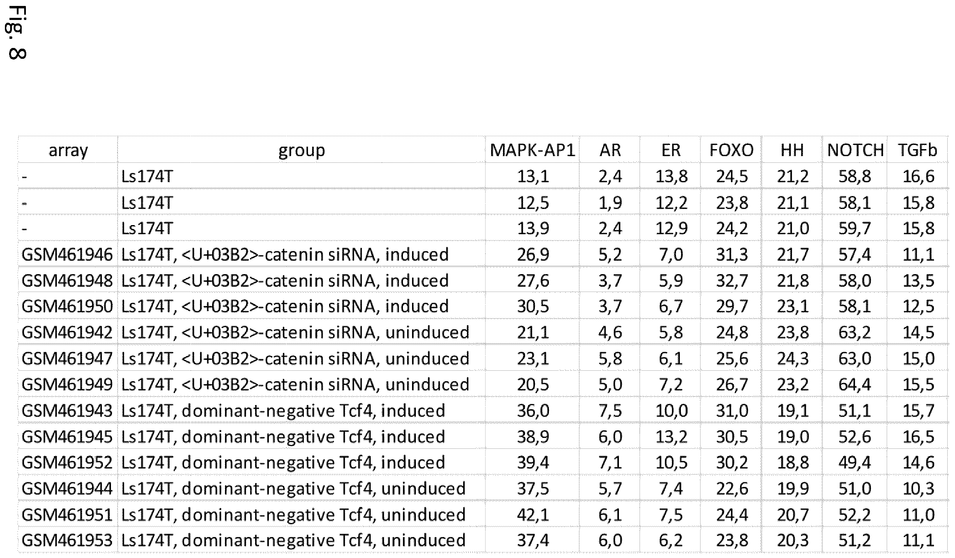

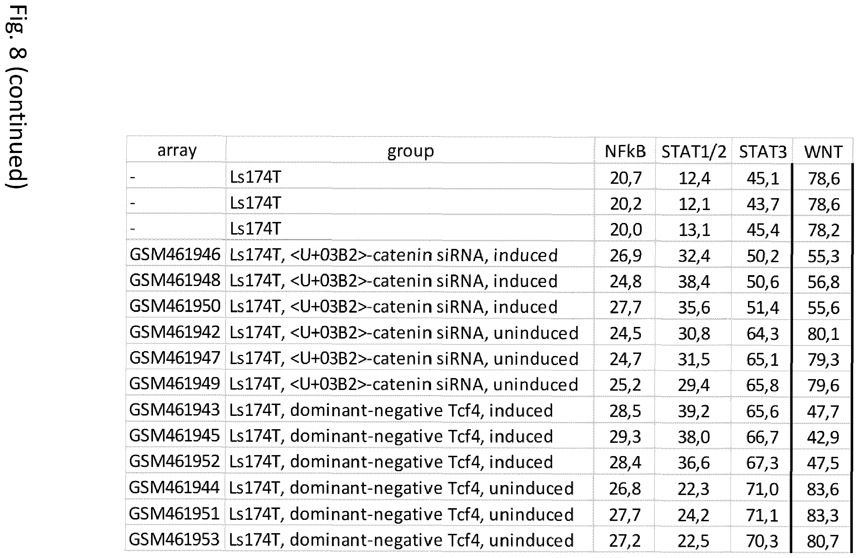

Other conditions that are envisioned that can be tested using the pathway fingerprint are the effect on cellular signaling pathway activity are for example: culture conditions (e.g. temperature, pH, salts or buffers), growth medium, factors included in the growth medium such as serum or growth factors, and cell attachment substrates used in cell culturing. The pathway fingerprint may be used to determine the effect of these different conditions on pathway activities, or to standardize protocols, or to compare or validate experimental result (e.g. by comparing the fingerprint of a control sample with a reference fingerprint). Additionally the effect of transfection with a DNA or RNA construct can be determined using the pathway fingerprint, as exemplified by the data represented by Fig. 8.

It is further envisioned that that the fingerprint may be used as a screening tool. For example compound may be tested for their potential pathway inhibiting or stimulating effect using the methods described herein. The advantage of the resented method using the fingerprint is that the effect on multiple pathways can be screened simultaneously, and moreover specificity of the compound can be assessed based on the determined pathway activities.

*C. Model system development*

Because the fingerprint provides information on the functional status of the cell by way of the most relevant signaling pathways regulating its functioning, the fingerprint may be used in the development of model systems. This may for example be useful for disease models, where experimental results rely on primary cultures of a patient, e.g. for testing potential treatments. The drawback of primary cultures is that they can be kept in culture for only a very brief period of time. It is thus envisioned that existing immortalized cell lines are screened based on a matching fingerprint with the fingerprints of the respective primary cell lines, so that the immortalize cell lines can be used as a substitute disease model.

Therefore, in an embodiment, the testing is used to:

- determine the passage number of the cell line; or
- determine whether the cell line is within an acceptable range of passages; or
- to confirm or identify the identity of a cell line; or

- to determine the effect of a condition on a cell line; or
- to perform a quality control on the cell line; or
- to compare different samples; or
- to select for a suitable cell model system to mimic a disease.

*5. Product and uses thereof*

In a second aspect the invention relates to a product for determining the cellular signaling pathway activities of four or more cellular signaling pathways selected from the ER, AR, PI3K/FOXO, MAPK-AP1, HH, NOTCH, TGFbeta, NFkB, STAT1/2, STAT3 and WNT pathways, wherein the pathway activity is based on the expression levels of three or more target genes for each cellular signaling pathway, wherein the product comprises primers and optionally probes for determining the expression levels of said three or more target genes for each cellular signaling pathway, and wherein the three or more genes of the ER pathway are selected from the group consisting of CDH26, SGK3, PGR, GREB1, CA12, XBP1, CELSR2, WISP2, DSCAM, ERBB2, CTSD, TFF1 and NRIP1, optionally further comprising one or more target genes selected from AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;
the three or more genes of the AR pathway are selected from the group consisting of KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally further comprising one or more target genes selected from APP, NTS, PLAU, CDKN1A, DRG1, FGF8, IGF1, PRKACB, PTPN1, SGK1 and TACC2;
the three or more genes of the PI3K/FOXO pathway are selected from the group consisting of AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10, optionally further comprising one or more target genes selected from ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4, optionally further comprising one or more target genes selected from ATG14, BIRC5, IGFBP1, KLF2, KLF4, MYOD1, PDK4, RAG1, RAG2, SESN1, SIRT1, STK11 and TXNIP;
the three or more genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;
the three or more genes of the HH pathway are selected from the group consisting of GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally further comprising one or more target genes selected from BCL2, FOXA2, FOXF1, H19, HHIP, IL1R2, JAG2, JUP, MIF, MYLK, NKX2.2, NKX2.8, PITRM1 and TOM1;
the three or more genes of the NOTCH pathway are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more genes of the TGFbeta pathway are selected from the group consisting of ANGPTL4, CDC42EP3, CDKN1A, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;
the three or more genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;
the three or more genes of the STAT1/2 pathway are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1, and USP18, or the three or more genes of the STAT1/2 pathway are selected from the group consisting of GBP1, IRF9, STAT1, TAP1, ISG15, APOL1, IRF1, IRF7, IFI6, IFITM1, USP18, CXCL9, OAS1, APOL2, IFIT2 and LY6E;

the three or more genes of the STAT3 pathway are selected from the group consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;

the three or more genes of the WNT pathway are selected from the group consisting of KIAA1199, AXIN2, RNF43, TBX3, TDGF1, SOX9, ASCL2, IL8, SP5, ZNRF3, KLF6, CCND1, DEFA6 and FZD7, optionally further comprising one or more target genes selected from NKD1, OAT, FAT1, LEF1, GLUL, REG1B, TCF7L2, COL18A1, BMP7, SLC1A2, ADRA2C, PPARG, DKK1, HNF1A and LECT2.

In a preferred embodiment, the product further comprises primers and optionally probes for determining the expression levels of one or more, e.g. one, two, three, four, five, six, seven, eight, nine, ten, eleven, twelve or more housekeeping genes. The housekeeping genes may be selected from the group consisting of ACTB, ALAS1, B2M, EEF1A1 POLR2A, PUM1, RPLP0, TBP, TPT1 and TUBA1B.

When used herein, "primers for determining the expression level" refers to primers which may be used in the amplification step for the detection of the expression levels. For example the primers may be primers suitable for qPCR. Typically the primers will be a forward and a reverse primer. Preferably the primers amplify a short sequence, e.g. about 50 to 500 nucleotides. Preferably the primers amplify an exon spanning sequence to avoid amplification of genomic DNA sequences.

When used herein, "probes for determining the expression level" refers to probes which may be used in the detection step for the detection of the expression levels. For example the probes may probes suitable for qPCR. Suitable probes for qPCR are known to the skilled person and commercially available.

It is envisioned the product as described herein can be advantageously used to determine five or more, e.g. five, six, seven, eight, nine, ten or more, cellular signaling pathway activities. The product comprises primers and optionally probes for the specific amplification and optionally detection of the three or more target genes, and is therefore for example suitable for quantification of expression levels of the target genes using quantitative or semi quantitative PCR. Methods for designing primers and probes for the quantitative detection of expression levels are well known in the art. For example qPCR can be used. The genes named here are listed under their default name. Their sequences can be retrieved from a public database such NCBI genes or genome browsers such as Ensembl or the UCSC genome browser. In a preferred embodiment the product comprises the probes. In a further preferred embodiment the product is for performing quantitative PCR. The product may further comprise additional reagents suitable for the purpose of amplification and optionally detection of a target gene, such buffers and/or enzymes and/or other reagents which can be used in the amplification and/or detection, such as dNTP, MgC12 solution, Reverse Transcriptase and/or polymerase. The product may also be a kit. The kit may additionally comprise buffers and/or enzymes and/or other reagents which can be used in the detection, such as dNTP, MgCl2 solution, Reverse Transcriptase and/or polymerase.

In a third aspect the invention relates to the use of the product according to second aspect of the invention. Particularly, the invention relates to use of the product according to the second aspect of the invention in the method according to the first aspect of the invention.

In a fourth aspect the invention relates to the use of the product according to the second aspect of the invention in:

- determining the passage number of the cell line; or
- determining whether the cell line is within an acceptable range of passages; or
- confirming or identifying the identity of a cell line; or
- determining the effect of a condition on a cell line; or
- performing a quality control on the cell line; or
- comparing different samples; or
- to select for a suitable cell model system to mimic a disease.

Figure legends

Fig. 1. Technical reproducibility of the pathway assays. Pathway activity scores of the MDA-MB-231 cell line with induced FOXO-3A construct and the MDA-MB-231 control cell line. Depicted are the ER, AR, PI3K, MAPK-AP1, HH, NOTCH and TGFbeta cellular signaling pathway activities.

Fig. 2. Ovarian cancer cell line A2780 was cultured under standard conditions and RNA was extracted from cells at passages px+5 and px+18. Depicted are the ER, AR, PI3K, MAPK-AP1, HH, NOTCH and TGFbeta cellular signaling pathway activities for passage 5 and 18. No changes are observed in pathway activity scores for the AR, ER, Notch and TGFβ.

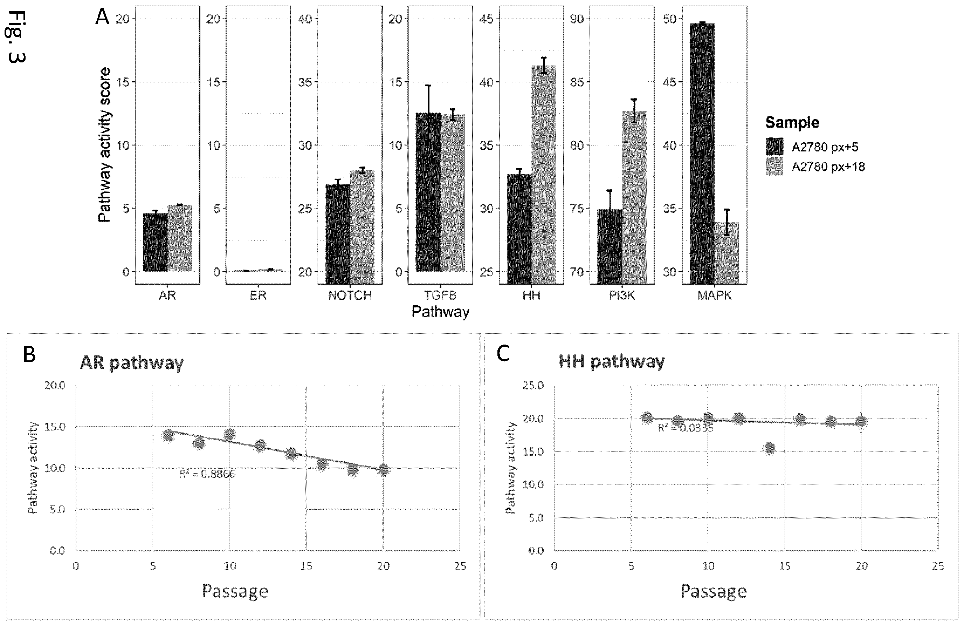

Fig. 3. T47D breast cancer cell line was cultured under standard conditions. Cells were passaged 3 times a week. 3A) Depicted are the ER, AR, PI3K, MAPK-AP1, HH, NOTCH and TGFbeta cellular signaling pathway activities for passage 5 and 18. 3B) Depicted is AR pathway activity over time. 3C) Depicted is HH pathway activity over time.

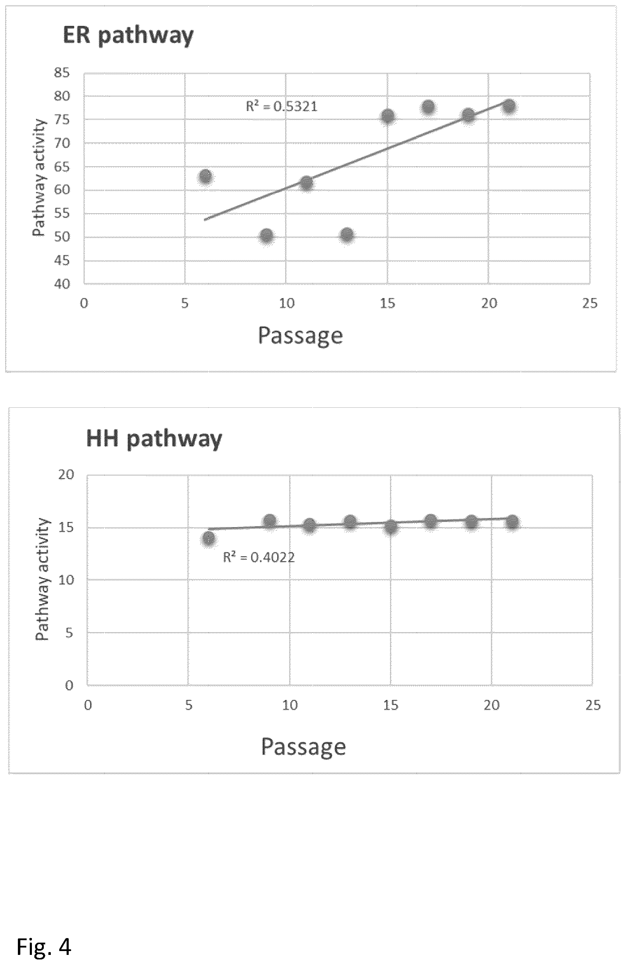

Fig. 4. Breast cancer cell line MCF7 was cultured under standard conditions, and RNA was isolated at indicated passages (6,9,11,13,15,17,19,21). Depicted are ER and Hedgehog (HH) cellular signaling pathway activity over the different passages.

Fig. 5. (A-F) different cell lines cultured in different labs. Each cell lines has been used in different studies as indicated by the different dataset GSE numbers. In some cases the commonly used solvent ETOH has been used as a vehicle control in very low concentrations. Depicted are the MAPK-AP1, AR, ER, FOXO, HH, NFkB, NOTCH, JAK-STAT1/2, JAK-STAT3, TGFbeta and WNT cellular signaling pathway activities.

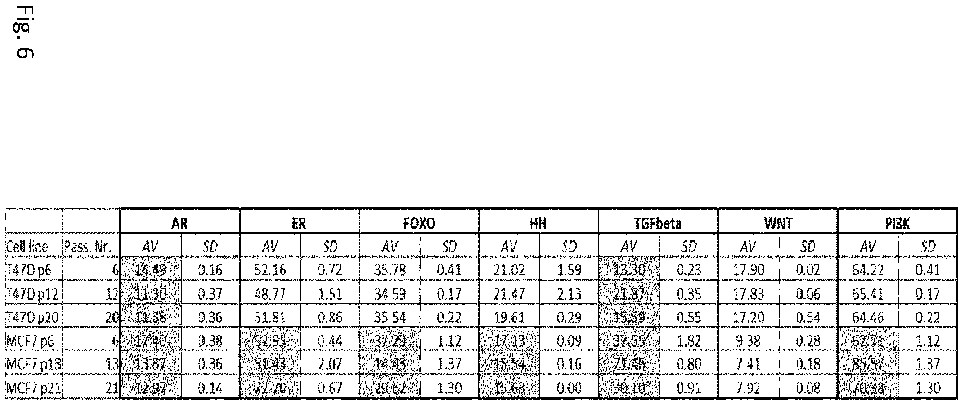

Fig. 6. Cellular signaling pathway activities for passages 6, 12 and 20 (T47D) or 6, 13, 21 (MCF7) were determined in triplicate. Indicated are the average pathway activity values and standard deviations for the ER, AR, PI3K, MAPK-AP1, HH, NOTCH and TGFbeta cellular signaling pathways.

Fig. 7. (A) Depicted is the experimental procedure for determining the pathway activities for cells treated with heat inactivated Fetal Brain Serum (FBS) or with non-heat inactivated FBS. (B) Depicted are the pathway activity values and standard deviations for the ER, AR, PI3K, MAPK-AP1, HH, NOTCH and TGFbeta cellular signaling pathways obtained in the heat inactivated vs non-heat inactivated FBS experiment.

Fig. 8. Comparison of non-transfected cells with cells transfected with either inducible beta-catenin siRNA or inducible dominant negative TCF4. Depicted are the AR, TGFBeta, WNT, JAK-STAT1/2, JAK-STAT3, NFkB, PI3K, MAPK, HH, NOTCH pathway activities for the non-transfected cell and the transfected cell in induced or non-induced state.

Fig. 9. Comparison of pathway activities in high or low confluent cell samples. (A) Depicted are exemplary samples used for high confluency or low confluency. (B) Graphical representation of pathway activities in high and low confluent cell samples. Depicted are the MAPK-AP1, AR, ER, FOXO, HH, NOTCH, TGFbeta and PI3K (inverse of FOXO) cellular signaling pathway activities. Left bars represent high confluency samples, right bars represent low confluency samples. (C) Depicted are the average pathway activities and the standard deviations. The experiment was performed in triplicate.

## Examples

### Analysis of Affymetrix microarray datasets

Using the developed signaling pathway tests for AR, TGFBeta, WNT, JAK-STAT1/2, JAK-STAT3, NFkB, PI3K, MAPK, HH, NOTCH pathways, public Affymetrix HG-U133Plus2.0 microarray expression datasets from preclinical and clinical studies (deposited in the GEO database) were analyzed and log2odds pathway activity scores calculated. Analyzed public datasets are indicated with their GSE number, and individual samples with their GSM number.

### Microarray data quality control

Quality control (QC) was performed on Affymetrix data of each individual sample. In summary, QC parameters include: the average value of all probe intensities, presence of negative or extremely high (> 16-bit) intensity values, poly-A RNA (sample preparation spike-ins) and labelled cRNA (hybridization spike ins) controls, *GAPDH* and *ACTB* 3'/5' ratio, centre of intensity and values of positive and negative border controls determined by affyQCReport package in R, and an RNA degradation value determined by the AffyRNAdeg function from the Affymetrix package in R.

### Reproducibility of the pathway assays

MDA-MB-231 cell line was cultured using standard conditions (DMEM + 10%FBS + 1% Glx +1%p/s) and treated with doxyclycline. This experiment was repeated 5 times. As shown in Fig. 1 below, maximal standard deviation was 3.3 pathway activity scores for the treated cells. These experiments show that the technical reproducibility of the pathway assays was robust.

Fig. 3 shows the T47D cell line which was cultured under standard culture conditions (RPMI1640 + 10%FBS + 1%p/s) Pathway activity was measured from passage 6 until passage 20. No changes are observed in pathway activity scores for the all pathways except for AR in which a decrease was shown from 14.2 normalized pathway in passage 6 towards a score of 10.0 in passage 20.

Fig. 4 shows the MCF-7 cell line which was cultured under standard culture conditions (RPMI1640 + 10%FBS + 1%Glx + 1%p/s) Pathway activity was measured from passage 6 until passage 21.). Hedgehog (HH) pathway activity remained stable over the different passages, whereas the estrogen receptor (ER) pathway activity increased with passaging (corr. 0.54).

Fig. 6 shows T47D and MCF7 cell lines at several passage numbers. The pathway activities were determined in triplicate to see variation in the obtained pathway activities. As can be seen from the standard deviations displayed in the table, very low variation was observed.

Proof points from public geo datasets

We collected the cell line data from several geo datasets and compared the pathway scores to see the variation between the cell lines. Pathway results are shown in fig. 5.

Cell lines which were included: T47D (5A), CACO-2 (5B), U937 (5C), MDA-MB-231 (5D), BT474 (5E) and IPS cell lines (5F).

As shown in the examples below large variation in pathway scores were shown for cell line between studies, but not within a study. This variation can be explained by the difference in culturing conditions and sample preparation. Studies are indicated by the different GSE numbers. For example in the cell line data of T47D, GSE34211 large differences are shown compared to the GSE32670, especially for the pathways MAPK-AP1, ER and JAK-STAT3.

Selecting cell lines which are representative for a disease (model) of interest.

Cell lines have the advantage above animal models of being derived from patients and are more easily to manipulate in the laboratory. However, these cell line model systems cannot completely replicate the environment of human tumors or diseased tissue and, even within the same cancer cell line model, data are often irreproducible between laboratories [21]. This may mean that a cell line is being used that is potentially not representative for the disease (or subtype).

To overcome this, one may apply cell line fingerprinting. This can for example be performed based on DNA to match the disease with the cell line. However, not all DNA mutations will lead to phenotypic changes which influences/determines the phenotype of the cancer or the disease.

By using finger-printing based on signaling transduction pathways it would able to select, based on extensive quantitative characterization of the cellular phenotype, the most representative cell line to a specific subtype of cancers, diseases or for example subpopulations of patients. Selecting disease specific cell lines which are representative for a target population could improve e.g. insights in disease mechanisms and response (prediction) towards drugs.

For many diseases there is no representative immortalized cell model, and much research is currently performed using primary cultures from biopsy material. These primary cell cultures have the disadvantage of only limited doublings and large sample patient variation.

The fingerprinting method based on signaling transduction pathways enables selecting immortalized cell lines which represents a disease subtype of interest. In this manner, cancer cell lines with the same pathway activity profile as the disease of interest could be used as a model (e.g. cancer cell line which is representative based on the signaling transduction pathway activity for a non-cancerous disease.)

To do so, first the fingerprinting has to be performed on the affected tissue material of the individual patient or patient population of interest. To accomplish this fingerprinting, pathway activity scores, divided into hormonal (H) pathways (ER, AR, PR), growth factor (GF) pathways (PI3K, MAPK-AP1, JAK-STAT3), immune (I) pathways (JAK-STAT1/2, NFkB), and developmental (D) pathways (Wnt, HH, Notch, TGFbeta) can be measured. By comparing this to a database with pathway score fingerprints of cell lines, a cell line can be selected with the closest fingerprint, to be used as model to represent the disease of interest.

This method is not limited towards a single cell line but can also be applied to a co-culture, 2D, 3D culture, and organoids. Furthermore, when a cell line phenotype does not represents the disease of interest, the cell line can be steered towards the disease phenotype by for example stimulators or inhibitors. The signaling transduction pathways can measure this effect and determine if the phenotype is now matching the disease (subtype).

Replicate data confirms low variability on pathway activity in technical triplicates

To verify low variability of determined pathway activities within samples (and thus low variability of fingerprint data within similar samples), the pathway activities for the cell passage experiment was obtained in triplicate. The average pathway activities are plotted in Fig.6, together with the standard deviations. As can be derived from the low standard deviations, very low variability is observed for the calculated pathway activities within the same sample.

Heat inactivated vs non heat inactivated FBS

Cells were first cultured for 4 passages in culture medium with 10% heat inactivated or non-heat inactivated FBS. After this cells are cultured for 7 days with new conditions; 10% heat inactivated FBS;10% non-heat inactivated FBS or 0,5% heat inactivated FBS. The experimental setup is also summarized in Fig.7A. Conditions also shown in table in column "Group" in Fig.7B.

Conclusions M059K:

10% Heat inactivation of FBS in M059K culture leads to increase in pathway activity of ER, FOXO, HH and decrease of TGHFbeta and PI3K.
0.5% heat inactivation FBS compated to 10% inactivated FBS resulted in lower TGFbeta and WNT activity.

Conclusions U87MG:
Largest effect seen (TGFbeta) when switching from heat inactivated towards non heat inactivated 10%FBS medium.

Use of pathway fingerprint to verify experimental setup.

In the next experiment, the pathway fingerprint is used to verify an experimental setup. Experiments in cell lines are often performed by introducing either transiently or stably a DNA construct, for example to introduce a mutant gene to mimic a disease. The pathway fingerprint can be used to verify if the expected cellular signaling pathways are affected while other pathways remain unaffected. This is particularly true for inducible systems, where no pathway activity changes are expected when the system is not induced. To test this hypothesis, pathway activities were determined on public datasets and cell culture data.

In the experiment non transfected Ls147T cells were used, and compared to Ls147T cells expressing either an inducible beta-catenin siRNA construct or an inducible dominant negative TCF4 construct. No changes in the pathway activities are expected in the non-transfected cells vs. the transfected but not induced cells. Downregulation of beta-catenin by siRNA is expected to reduce WNT cellular signaling activity, similarly expression of dominant negative TCF4 is expected to lead to reduced WNT signaling pathway activity. All experiments are performed in triplicate and pathway activities were determined for MAPK-AP1, AR, ER, FOXO, HH, NOTCH, TGFbeta, NFkB, JAK-STAT1/2, JAK-STAT3 and WNT. The results are shown in Fig. 8.

As expected WNT activity was reduced when beta catenin siRNA or dominant negative TCF4 was induced, but not non-induced control samples. The pathway fingerprint however further reveals which pathways are affected by transfection of constructs (pathway activities deviating in non-induced samples vs non transfected samples) and which other pathways may by directly or indirectly influenced by the induced constructs (by comparing the non-induced with the induced samples).

Influence of confluency on pathway activities.

In cell line experiments it may occur that differing samples with different confluencies are compared. It was hypothesized that high and low confluency may effect pathway activities and thus affect experimental results. To test this hypothesis, samples were collected from high and low confluent cell cultures in triplicate, and pathway activities were determined. The experimental results are displayed in Fig.9. A statistical difference in pathway activity was found for ER, FOXO and PI3K cellular signaling pathway activity. It is noted that PI3K activity is calculated as the universe of FOXO activity. This confirms that indeed pathway activity is affected by confluency. The pathway fingerprint may be used to correct for differences in confluency, or to establish that samples have similar confluencies (as determined by pathway activities).

REFERENCES

[1] C. A. Chacón-Martínez, J. Koester, and S. A. Wickström, "Signaling in the stem cell niche: regulating cell fate, function and plasticity," Development, vol. 145, no. 15, p. dev165399, Aug. 2018.
[2] L.C. Cantley, T. Hunter, R. Sever, J. W. Thorner, Signal transduction: principles, pathways, and processes. Cold Spring Harbor, New York : Cold Spring Harbor Laboratory Press, 2014. Cold Spring Harbor, New York : Cold Spring Harbor Laboratory Press, 2014.
[3] C. Mummery, A. van de Stolpe, B. Roelen, and H. Clevers, Stem Cells: Scientific Facts and Fiction. Academic Press, 2014.

[4] J. S. L. Yu and W. Cui, "Proliferation, survival and metabolism: the role of PI3K/AKT/mTOR signalling in pluripotency and cell fate determination," Development, vol. 143, no. 17, pp. 3050-3060, Sep. 2016.

[5] J.-F. Arnal et al., "Membrane and Nuclear Estrogen Receptor Alpha Actions: From Tissue Specificity to Medical Implications," Physiol. Rev., vol. 97, no. 3, pp. 1045-1087, 01 2017.

[6] M. Kono, T. Fujii, B. Lim, M. S. Karuturi, D. Tripathy, and N. T. Ueno, "Androgen Receptor Function and Androgen Receptor-Targeted Therapies in Breast Cancer: A Review," JAMA Oncol, vol. 3, no. 9, pp. 1266-1273, Sep. 2017.

[7] C. Siebel and U. Lendahl, "Notch Signaling in Development, Tissue Homeostasis, and Disease," Physiol. Rev., vol. 97, no. 4, pp. 1235-1294, 01 2017.

[8] M. Burotto, V. L. Chiou, J.-M. Lee, and E. C. Kohn, "The MAPK pathway across different malignancies: a new perspective," Cancer, vol. 120, no. 22, pp. 3446-3456, Nov. 2014.

[9] E. Pardali, G. Sanchez-Duffhues, M. C. Gomez-Puerto, and P. Ten Dijke, "TGF-β-Induced Endothelial-Mesenchymal Transition in Fibrotic Diseases," Int J Mol Sci, vol. 18, no. 10, Oct. 2017.

[10] G. Sánchez-Duffhues, A. Garcia de Vinuesa, and P. Ten Dijke, "Endothelial-to-mesenchymal transition in cardiovascular diseases: Developmental signaling pathways gone awry," Dev. Dyn., vol. 247, no. 3, pp. 492-508, 2018.

[11] F. A. High and J. A. Epstein, "The multifaceted role of Notch in cardiac development and disease," Nat. Rev. Genet., vol. 9, no. 1, pp. 49-61, Jan. 2008.

[12] G. Sanchez-Duffhues, V. Orlova, and P. Ten Dijke, "In Brief: Endothelial-to-mesenchymal transition," J. Pathol., vol. 238, no. 3, pp. 378-380, Feb. 2016.

[13] M. Bienz and H. Clevers, "Linking colorectal cancer to Wnt signaling," Cell, vol. 103, no. 2, pp. 311-320, Oct. 2000.

[14] S. S. Karhadkar et al., "Hedgehog signalling in prostate regeneration, neoplasia and metastasis," Nature, vol. 431, no. 7009, pp. 707-712, Oct. 2004.

[15] J. Taipale and P. A. Beachy, "The Hedgehog and Wnt signalling pathways in cancer," Nature, vol. 411, no. 6835, pp. 349-354, May 2001.

[16] D. Hanahan and R. A. Weinberg, "Hallmarks of cancer: the next generation," Cell, vol. 144, no. 5, pp. 646-674, Mar. 2011.

[17] A. van de Stolpe, "On the origin and destination of cancer stem cells: a conceptual evaluation," Am J Cancer Res, vol. 3, no. 1, pp. 107-116, 2013.

[18] "https://www.cancer.gov/about-cancer/treatment/types/targeted-therapies/targeted-therapies-fact-sheet."

[19] T. A. Baudino, "Targeted Cancer Therapy: The Next Generation of Cancer Treatment," Curr Drug Discov Technol, vol. 12, no. 1, pp. 3-20, 2015.

[20] Y. K. Chae et al., "Path toward Precision Oncology: Review of Targeted Therapy Studies and Tools to Aid in Defining 'Actionability' of a Molecular Lesion and Patient Management Support," Mol Cancer Ther, vol. 16, no. 12, pp. 2645-2655, Dec. 2017.

[21] U. Ben-David et al., "Genetic and transcriptional evolution alters cancer cell line drug response," Nature, vol. 560, no. 7718, pp. 325-330, 2018.

**Claims**

1. A method for testing a cell line based on a sample obtained from said cell line, wherein the method is based on a cellular signaling pathway fingerprint, wherein the fingerprint is defined as a plurality of numerical values representing distinct cellular signaling pathway activities in a sample, and wherein the testing comprises comparing the cellular signaling pathway fingerprint to a reference cellular signaling pathway fingerprint.

2. Method according to claim 1, wherein the testing further comprises:

   - determining the expression levels of at least three target genes for each cellular signaling pathway in the fingerprint.

3. Method according to claim 2, wherein the expression levels are based on mRNA isolated from the sample.

4. Method according to claim 3, wherein the testing further comprises isolating mRNA from the sample.

5. Method according to any one of the preceding claims, wherein the fingerprint comprises four or more cellular signaling pathways selected from the ER, AR, PI3K/FOXO, MAPK-AP1, HH, NOTCH, TGFbeta, NFkB, STAT1/2, STAT3 and WNT pathways.

6. Method according to any one of the preceding claims, wherein:

the three or more genes of the ER pathway are selected from the group consisting of CDH26, SGK3, PGR, GREB1, CA12, XBP1, CELSR2, WISP2, DSCAM, ERBB2, CTSD, TFF1 and NRIP1, optionally further comprising one or more target genes selected from AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;

the three or more genes of the AR pathway are selected from the group consisting of KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally further comprising one or more target genes selected from APP, NTS, PLAU, CDKN1A, DRG1, FGF8, IGF1, PRKACB, PTPN1, SGK1 and TACC2;

the three or more genes of the PI3K/FOXO pathway are selected from the group consisting of AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10, optionally further comprising one or more target genes selected from ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4, optionally further comprising one or more target genes selected from ATG14, BIRC5, IGFBP1, KLF2, KLF4, MYOD1, PDK4, RAG1, RAG2, SESN1, SIRT1, STK11 and TXNIP;

the three or more genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;

the three or more genes of the HH pathway are selected from the group consisting of GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally further comprising one or more target genes selected from BCL2, FOXA2, FOXF1, H19, HHIP, IL1R2, JAG2, JUP, MIF, MYLK, NKX2.2, NKX2.8, PITRM1 and TOM1;

the three or more genes of the NOTCH pathway are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;

the three or more genes of the TGFbeta pathway are selected from the group consisting of ANGPTL4, CDC42EP3, CDKN1A, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;

the three or more genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;

the three or more genes of the STAT1/2 pathway are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1, and USP18, or the three or more genes of the STAT1/2 pathway are selected from the group consisting of GBP1, IRF9, STAT1, TAP1, ISG15, APOL1, IRF1, IRF7, IFI6, IFITM1, USP18, CXCL9, OAS1, APOL2, IFIT2 and LY6E;

the three or more genes of the STAT3 pathway are selected from the group consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;

the three or more genes of the WNT pathway are selected from the group consisting of KIAA1199, AXIN2, RNF43, TBX3, TDGF1, SOX9, ASCL2, IL8, SP5, ZNRF3, KLF6, CCND1, DEFA6 and FZD7, optionally further comprising one or more target genes selected from NKD1, OAT, FAT1, LEF1, GLUL, REG1B, TCF7L2, COL18A1, BMP7, SLC1A2, ADRA2C, PPARG, DKK1, HNF1A and LECT2.

7. Method according to any one of the preceding claims, wherein the testing is used to:

- determine the passage number of the cell line; or
- determine whether the cell line is within an acceptable range of passages; or
- to confirm or identify the identity of a cell line; or
- to determine the effect of a condition on a cell line; or
- to perform a quality control on the cell line; or
- to compare different samples; or
- to select for a suitable cell model system to mimic a disease.

8. A product for determining the cellular signaling pathway activities of four or more cellular signaling pathways selected from the ER, AR, PI3K/FOXO, MAPK-AP1, HH, NOTCH, TGFbeta, NFkB, STAT1/2, STAT3 and WNT pathways, wherein the pathway activity is based on the expression levels of three or more target genes for each cellular signaling pathway, wherein the product comprises primers and optionally probes for determining the expression levels of said three or more target genes for each cellular signaling pathway, and wherein
the three or more genes of the ER pathway are selected from the group consisting of CDH26, SGK3, PGR, GREB1, CA12, XBP1, CELSR2, WISP2, DSCAM, ERBB2, CTSD, TFF1 and NRIP1, optionally further comprising one or more target genes selected from AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;
the three or more genes of the AR pathway are selected from the group consisting of KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally further comprising one or more target genes selected from APP, NTS, PLAU, CDKN1A, DRG1, FGF8, IGF1, PRKACB, PTPN1, SGK1 and TACC2;
the three or more genes of the PI3K/FOXO pathway are selected from the group consisting of AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2 and TNFSF10, optionally further comprising one or more target genes selected from ATP8A1, C10orf10, CBLB, DDB1, DYRK2, ERBB3, EREG, EXT1, FGFR2, IGF1R, IGFBP1, IGFBP3, LGMN, PPM1D, SEMA3C, SEPP1, SESN1, SLC5A3, SMAD4 and TLE4, optionally further comprising one or more target genes selected from ATG14, BIRC5, IGFBP1, KLF2, KLF4, MYOD1, PDK4, RAG1, RAG2, SESN1, SIRT1, STK11 and TXNIP;
the three or more genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;
the three or more genes of the HH pathway are selected from the group consisting of GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally further comprising one or more target genes selected from BCL2, FOXA2, FOXF1, H19, HHIP, IL1R2, JAG2, JUP, MIF, MYLK, NKX2.2, NKX2.8, PITRM1 and TOM1;
the three or more genes of the NOTCH pathway are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more genes of the TGFbeta pathway are selected from the group consisting of ANGPTL4, CDC42EP3, CDKN1A, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;

the three or more genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;

the three or more genes of the STAT1/2 pathway are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, and ZNRF3, preferably, from the group consisting of: IRF1, IRF7, IRF8, IRF9, OAS1, PDCD1, ST13, STAT1, and USP18, or the three or more genes of the STAT1/2 pathway are selected from the group consisting of GBP1, IRF9, STAT1, TAP1, ISG15, APOL1, IRF1, IRF7, IFI6, IFITM1, USP18, CXCL9, OAS1, APOL2, IFIT2 and LY6E;

the three or more genes of the STAT3 pathway are selected from the group consisting of AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JunB, PTGS2, STAT1, TNFRSF1B, and ZEB1;

the three or more genes of the WNT pathway are selected from the group consisting of KIAA1199, AXIN2, RNF43, TBX3, TDGF1, SOX9, ASCL2, IL8, SP5, ZNRF3, KLF6, CCND1, DEFA6 and FZD7, optionally further comprising one or more target genes selected from NKD1, OAT, FAT1, LEF1, GLUL, REG1B, TCF7L2, COL18A1, BMP7, SLC1A2, ADRA2C, PPARG, DKK1, HNF1A and LECT2.

9. Use of the product according to claim 8 in the method according to any one of claims 1 to 7.

10. Use of the product according to claim 8 in:

   - determining the passage number of the cell line; or
   - determining whether the cell line is within an acceptable range of passages; or
   - confirming or identifying the identity of a cell line; or
   - determining the effect of a condition on a cell line; or
   - performing a quality control on the cell line; or
   - comparing different samples
   - to select for a suitable cell model system to mimic a disease

EP 4 039 825 A1

**Fig. 1**

| Sample | Number of replicates | ER Score ± SD | AR Score ± SD | PI3K Score ± SD |
|---|---|---|---|---|
| MDA-MB-231 cells 18 hrs doxycycline induced FOXO | 5 | 8.4 ± 3.3 | 17.7 ± 0.9 | 31.8 ± 3.9 |
| MDA-MB-231 cells untreated | 5 | 7.0 ± 1.1 | 17.2 ± 0.7 | 73.7 ± 0.9 |

| Sample | MAPK Score ± SD | HH Score ± SD | NOTCH Score ± SD | TGFβ Score ± SD |
|---|---|---|---|---|
| MDA-MB-231 cells 18 hrs doxycycline induced FOXO | 65.2 ± 1.1 | 26.6 ± 2.5 | 14.6 ± 1.3 | 22.2 ± 1.4 |
| MDA-MB-231 cells untreated | 61.6 ± 1.4 | 31.4 ± 2.3 | 16.7 ± 1.2 | 20.3 ± 1.4 |

Fig. 2

Fig. 3

Fig. 4

Fig. 5

A

| group | dataset | QC.passed | QC.man.p | AP1 | | AP1-B | AR | |
|---|---|---|---|---|---|---|---|---|
| | | | | log2odds. | log2odds. | log2odds. | log2odds. | log2odds. |
| untreated control (3 hours post chang | GSE32670 | FALSE | | -11.7 | -9.7 | -3.0 | -12.2 | -10.5 |
| untreated control (3 hours post chang | GSE32670 | FALSE | | -11.5 | -8.9 | -2.0 | -13.1 | -10.4 |
| treated for 3 hours with 0.1% Ethanol | GSE32670 | FALSE | | -11.9 | -9.2 | -0.5 | -12.8 | -10.8 |
| breast | GSE34211 | FALSE | | -4.6 | -5.3 | -0.5 | -11.3 | -9.7 |
| breast | GSE34211 | FALSE | | -6.6 | -5.7 | -1.2 | -10.9 | -8.9 |
| breast | GSE34211 | FALSE | | -4.8 | -5.7 | -0.4 | -12.0 | -10.0 |
| AZ GENE EXPRESSION | GSE57083 | FALSE | | -10.2 | -6.5 | 1.8 | -12.6 | -9.8 |
| T47D; EtOH | GSE60882 | TRUE | TRUE | -11.7 | -9.7 | -3.0 | -12.2 | -10.5 |
| T47D | GSE41445 | TRUE | | -11.7 | -6.8 | 0.4 | -11.8 | -10.6 |
| T47D | GSE41445 | FALSE | TRUE | -12.0 | -7.2 | 0.3 | -10.7 | -10.5 |
| T47D | GSE41445 | FALSE | TRUE | -11.6 | -6.4 | 0.5 | -11.5 | -10.2 |
| ATCC | GSE87006 | TRUE | | -11.1 | -9.7 | -2.0 | -8.6 | -8.6 |
| ATCC | GSE87008 | TRUE | TRUE | -11.1 | -9.7 | -2.0 | -8.6 | -8.6 |

EP 4 039 825 A1

Fig. 5 (continued)

A (continued)

| group | ER log2odds. | FOXO log2odds. | HH log2odds. | NFKB log2odds. | NOTCH log2odds. | STAT1/2-II log2odds.S | STAT1/2-I log2odds. | STAT3-E log2odds. | TGFB log2odds. | WNT log2odds. |
|---|---|---|---|---|---|---|---|---|---|---|
| untreated control (3 hours post chang | -4.5 | -3.0 | -11.3 | -16.8 | 6.7 | -8.5 | -10.5 | 0.6 | -16.9 | -19.6 |
| untreated control (3 hours post chang | 3.7 | -7.3 | -6.6 | -15.7 | 12.9 | -8.8 | -10.6 | -0.2 | -17.7 | -16.5 |
| treated for 3 hours with 0.1% Ethanol | 3.5 | -6.4 | -7.1 | -11.4 | 15.2 | -8.5 | -10.9 | -0.7 | -16.0 | -15.8 |
| breast | 13.2 | -6.5 | -6.9 | -16.0 | 15.9 | -8.8 | -11.1 | 7.6 | -18.1 | -20.3 |
| breast | 10.6 | -1.3 | -8.8 | -16.3 | 13.4 | -9.3 | -11.1 | 6.5 | -17.9 | -20.7 |
| breast | 12.9 | -8.2 | -7.3 | -14.4 | 14.3 | -9.1 | -10.3 | 7.2 | -19.0 | -20.1 |
| AZ GENE EXPRESSION | 9.4 | -5.8 | -0.1 | -9.9 | 17.5 | -8.3 | -9.7 | 6.6 | -13.4 | -20.3 |
| T47D; EtOH | -4.5 | -3.0 | -11.3 | -16.8 | 6.7 | -8.5 | -10.5 | 0.6 | -16.9 | -19.6 |
| T47D | 8.8 | 0.4 | -6.5 | -15.8 | 11.6 | -9.6 | -11.0 | 3.2 | -18.4 | -23.6 |
| T47D | 11.3 | 0.8 | -7.6 | -15.0 | 12.2 | -9.5 | -11.0 | 3.7 | -17.3 | -22.2 |
| T47D | 10.0 | 0.9 | -7.5 | -14.6 | 11.8 | -9.1 | -10.8 | 3.2 | -17.6 | -23.0 |
| ATCC | 9.1 | 2.0 | -10.6 | -17.2 | 8.1 | -8.9 | -10.6 | 0.5 | -16.6 | -23.1 |
| ATCC | 9.1 | 2.0 | -10.6 | -17.2 | 8.1 | -8.9 | -10.6 | 0.5 | -16.6 | -23.1 |

## Fig. 5 (continued)

B

| array | title | group | dataset | QC.passed | QC.man.p | AP1 log2odds. | AR log2odds. | ER log2odds. | FOXO log2odds. |
|---|---|---|---|---|---|---|---|---|---|
| GSM750842 | 3-week Caco-2 cells | colon carcinoma derived cell line | GSE30292 | FALSE | | -7.7 | -14.7 | -13.6 | -0.4 |
| GSM750843 | 3-week Caco-2 cells | colon carcinoma derived cell line | GSE30292 | FALSE | | -7.1 | -14.4 | -14.4 | -0.1 |
| GSM750844 | 3-week Caco-2 cells | colon carcinoma derived cell line | GSE30292 | FALSE | | -7.7 | -14.6 | -13.9 | -0.9 |
| GSM843481 | CACO2 replicate 1 | large_intestine | GSE34211 | TRUE | | 0.3 | -11.9 | -16.5 | -11.1 |
| GSM843482 | CACO2 replicate 2 | large_intestine | GSE34211 | FALSE | | -0.7 | -14.2 | -16.4 | -14.5 |
| EA06028_81819_ | CACO2 | GSK2 | WIMM | FALSE | | 0.8 | -18.1 | -19.3 | -8.6 |
| GSM1017460 | CACO2_3a | Caco-2 | GSE41445 | TRUE | | -9.3 | -12.3 | -18.0 | -11.3 |
| GSM1017461 | CACO2_3b | Caco-2 | GSE41445 | FALSE | TRUE | -8.5 | -12.4 | -18.0 | -10.9 |
| GSM1017462 | CACO2_3c | Caco-2 | GSE41445 | TRUE | | -7.1 | -11.1 | -17.9 | -10.1 |
| GSM224696 | Caco-2 Control-1 | medium alone | GSE8896 | TRUE | | 1.7 | -13.5 | -13.0 | -2.7 |
| GSM224697 | Caco-2 Control-2 | medium alone | GSE8896 | TRUE | | -5.1 | -13.1 | -13.5 | -4.9 |
| GSM224698 | Caco-2 Control-3 | medium alone | GSE8896 | TRUE | | 0.4 | -13.8 | -14.3 | -3.1 |

Fig. 5 (continued)

B (continued)

| array | title | HH log2odds. | NFKB log2odds. | NOTCH log2odds. | STAT1/2-II log2odds. | STAT1/2-I log2odds. | STAT3-E log2odds. | TGFB log2odds. | WNT log2odds. |
|---|---|---|---|---|---|---|---|---|---|
| GSM750842 | 3-week Caco-2 cells | -3.4 | -12.6 | 1.8 | -11.6 | -10.6 | -4.8 | -20.1 | -1.5 |
| GSM750843 | 3-week Caco-2 cells | -4.1 | -12.2 | 2.0 | -11.6 | -10.4 | -5.3 | -20.1 | -2.9 |
| GSM750844 | 3-week Caco-2 cells | -2.0 | -14.0 | 1.6 | -11.6 | -10.5 | -5.3 | -19.2 | -2.3 |
| GSM843481 | CACO2 replicate 1 | -8.8 | -15.2 | 10.5 | -8.3 | -11.4 | 5.1 | -13.4 | 10.8 |
| GSM843482 | CACO2 replicate 2 | -7.7 | -14.2 | 9.8 | -9.1 | -9.1 | 5.9 | -15.5 | 9.6 |
| EA06028_81819 | CACO2 | -1.6 | -6.2 | 10.2 | -11.2 | -11.8 | 4.9 | -19.8 | 2.8 |
| GSM1017460 | CACO2_3a | -7.3 | -14.7 | 4.0 | -10.7 | -10.9 | 2.9 | -20.1 | 2.6 |
| GSM1017461 | CACO2_3b | -7.8 | -15.0 | 3.9 | -10.5 | -11.2 | 2.9 | -20.0 | 1.4 |
| GSM1017462 | CACO2_3c | -9.3 | -15.4 | 5.4 | -10.9 | -11.3 | 3.5 | -19.6 | 4.3 |
| GSM224696 | Caco-2 Control-1 | -10.6 | -9.2 | 5.1 | -10.1 | -10.6 | 0.9 | -15.5 | -3.8 |
| GSM224697 | Caco-2 Control-2 | -11.3 | -8.2 | 3.7 | -10.4 | -11.0 | -1.6 | -17.8 | -4.0 |
| GSM224698 | Caco-2 Control-3 | -10.8 | -11.7 | 4.4 | -10.0 | -10.3 | 0.1 | -16.5 | -5.8 |

Fig. 5 (continued)

C

| array | title | group | dataset | QC.passed | QC.man.p | AP1 log2odds. |
|---|---|---|---|---|---|---|
| GSM421935 | U937 cells infected with empty pSICO-R vector - Replica 1 | U937 myelomonocytic cell line, control | GSE16798 | TRUE | | -3.3 |
| GSM421936 | U937 cells infected with empty pSICO-R vector - Replica 2 | U937 myelomonocytic cell line, control | GSE16798 | TRUE | | -2.9 |
| GSM460066 | U937T:PR no induction 48hr rep1 | U937T cells before induction | GSE18476 | TRUE | | -5.2 |
| GSM460067 | U937T:PR no induction 48hr rep2 | U937T cells before induction | GSE18476 | TRUE | | -5.6 |
| GSM460068 | U937T:PR no induction 48hr rep3 | U937T cells before induction | GSE18476 | TRUE | | -4.9 |
| GSM825118 | U937_DMSO | U937 cells treated with DMSO | GSE33358 | TRUE | | -1.3 |
| GSM1374975 | U937_ghp280606 | A2 GENE EXPRESSION | GSE57083 | FALSE | | -7.4 |
| GSM1633329 | U937_vec EtOH replicate 1 | U937_vec cells, 24 h EtOH treatment | AP1_calib | | TRUE | -6.2 |
| GSM1633329 | U937_vec EtOH replicate 1 | U937_vec cells, 24 h EtOH treatment | GSE66853 | TRUE | | -6.2 |
| GSM1633333 | U937_vec EtOH replicate 2 | U937_vec cells, 24 h EtOH treatment | AP1_calib | | TRUE | -6.5 |
| GSM1633333 | U937_vec EtOH replicate 2 | U937_vec cells, 24 h EtOH treatment | GSE66853 | TRUE | | -6.5 |
| GSM1633337 | U937_vec EtOH replicate 3 | U937_vec cells, 24 h EtOH treatment | AP1_calib | | TRUE | -6.8 |
| GSM1633337 | U937_vec EtOH replicate 3 | U937_vec cells, 24 h EtOH treatment | GSE66853 | TRUE | | -6.8 |

Fig. 5 (continued)

EP 4 039 825 A1

C (continued)

| title | AR log2odds. | ER log2odds. | FOXO log2odds. | HH log2odds. | NFKB log2odds. | NOTCH log2odds. | STAT1/2-II log2odds.S | STAT1/2-I log2odds. | STAT3-B log2odds. | TGFB log2odds. | WNT log2odds. |
|---|---|---|---|---|---|---|---|---|---|---|---|
| U937 cells infected with empty pSI | -10.9 | -11.9 | -5.6 | -11.4 | -2.0 | -0.1 | -7.3 | -9.3 | -7.8 | -11.6 | -17.9 |
| U937 cells infected with empty pSI | -11.1 | -12.4 | -5.5 | -11.2 | -2.3 | 0.1 | -7.4 | -9.2 | -8.1 | -11.7 | -17.8 |
| U937T:PR no induction 48hr rep1 | -14.1 | -10.4 | -12.0 | -8.4 | -11.0 | -5.3 | -7.8 | -8.8 | -11.1 | -20.9 | -14.4 |
| U937T:PR no induction 48hr rep2 | -14.2 | -10.5 | -11.0 | -8.8 | -9.8 | -5.5 | -7.3 | -8.6 | -10.7 | -21.2 | -15.2 |
| U937T:PR no induction 48hr rep3 | -15.2 | -12.2 | -13.8 | -9.5 | -9.8 | -6.5 | -7.4 | -7.9 | -12.4 | -21.7 | -14.7 |
| U937_DMSO | -9.9 | -12.4 | -5.1 | -7.2 | -2.5 | 1.1 | -8.1 | -10.2 | -9.2 | -14.7 | -17.2 |
| U937 ghp280606 | -11.5 | -5.6 | -4.9 | 5.0 | -4.8 | -1.5 | -9.3 | -10.9 | -8.3 | -12.9 | -12.7 |
| U937_vec EtOH replicate 1 | -10.3 | -12.0 | -6.4 | -5.9 | -4.1 | -3.0 | -8.7 | -9.5 | -8.6 | -16.6 | -17.5 |
| U937_vec EtOH replicate 1 | -10.3 | -12.0 | -6.4 | -5.9 | -4.1 | -3.0 | -8.7 | -9.5 | -8.6 | -16.6 | -17.5 |
| U937_vec EtOH replicate 2 | -10.5 | -12.1 | -6.0 | -5.3 | -3.0 | -3.1 | -9.2 | -10.2 | -9.5 | -17.5 | -18.2 |
| U937_vec EtOH replicate 2 | -10.5 | -12.1 | -6.0 | -5.3 | -3.0 | -3.1 | -9.2 | -10.2 | -9.5 | -17.5 | -18.2 |
| U937_vec EtOH replicate 3 | -10.5 | -13.8 | -5.9 | -3.6 | -4.4 | -3.2 | -9.0 | -9.5 | -8.3 | -17.0 | -18.1 |
| U937_vec EtOH replicate 3 | -10.5 | -13.8 | -5.9 | -3.6 | -4.4 | -3.2 | -9.0 | -9.5 | -8.3 | -17.0 | -18.1 |

Fig. 5 (continued)

D

| array | title | group | dataset | QC.passed | QC.man.p |
|---|---|---|---|---|---|
| A3753_01 | MDA-MB-231, control, replicate #1 | Cell line | A3753 | TRUE | TRUE |
| A3753_02 | MDA-MB-231, control, replicate #2 | Cell line | A3753 | FALSE | FALSE |
| A3753_03 | MDA-MB-231, control, replicate #3 | Cell line | A3753 | FALSE | FALSE |
| GSM320604 | MDA-MB-231 | ER negative | GSE12777 | FALSE | |
| GSM320604 | breast cancer cell line_MDA-MB-231 | Breast cancer | GSE12790 | TRUE | |
| GSM553882 | Breast cancer cell line MDA-MB-231 WT (expression data) | Breast cancer cell line | GSE22250 | TRUE | |
| GSM810964 | MDA-MB-231_control | untreated control (3 hours post change of medium) | GSE32670 | TRUE | |
| GSM810965 | MDA-MB-231_control (2) | untreated control (3 hours post change of medium) | GSE32670 | TRUE | |
| GSM978175 | MDA-MB-231 control ETOH (vehicle) | MDA-MB-231 cells, treated for 3 hours with 0.1% Ethanol | GSE39718 | TRUE | |
| GSM1178375 | MDA-MB-231T_originating cell line_R1 | MDA-MB-231T, originating cell line | GSE48433 | TRUE | |
| GSM1178376 | MDA-MB-231T_originating cell line_R2 | MDA-MB-231T, originating cell line | GSE48433 | TRUE | |
| GSM1357970 | Serum starved MDA-MB-231 cells, Rep1 | MDA-MB-231 cells, Serum starved | GSE56265 | TRUE | TRUE |
| GSM1357971 | Serum starved MDA-MB-231 cells, Rep2 | MDA-MB-231 cells, Serum starved | GSE56265 | FALSE | TRUE |
| GSM1374652 | MDA-MB-231 | AZ GENE EXPRESSION | GSE57083 | FALSE | |
| GSM1431022 | MDA-MB-231 cells siControl, biological replica A | MDA-MB-231 breast cancer cells | GSE59230 | TRUE | TRUE |
| GSM1431023 | MDA-MB-231 cells siControl, biological replica B | MDA-MB-231 breast cancer cells | GSE59230 | TRUE | TRUE |
| GSM1431024 | MDA-MB-231 cells siControl, biological replica C | MDA-MB-231 breast cancer cells | GSE59230 | TRUE | TRUE |
| GSM1431025 | MDA-MB-231 cells siControl, biological replica D | MDA-MB-231 breast cancer cells | GSE59230 | TRUE | TRUE |
| GSM1492781 | MDA-MB-231 control | MDA-MB-231; EtOH | GSE60882 | TRUE | TRUE |
| GSM1589153 | CellLine, MDA-MB-231 | Sample from breast cancer derived cell line MDA-MB-231 | GSE65194 | TRUE | |
| GSM1589797 | BA CellLine, MDA-MB-231 | Sample from breast cancer derived cell line MDA-MB-231 | GSE65212 | TRUE | |
| GSM887295 | MDA-MB-231 | ATCC | GSE36133 | TRUE | TRUE |
| GSM887295 | MDA-MB-231 | ATCC | GSE36139 | TRUE | |

33

Fig. 5 (continued)

## D (continued)

| title | AP1 log2odds. | AR log2odds. | ER log2odds. | FOXO log2odds. | HH log2odds. | NFKB log2odds. |
|---|---|---|---|---|---|---|
| MDA-MB-231, control, replicate #1 | 12.8 | -7.9 | -12.0 | -5.3 | -4.7 | -8.1 |
| MDA-MB-231, control, replicate #2 | 10.0 | -8.6 | -12.9 | -7.5 | -2.9 | -11.2 |
| MDA-MB-231, control, replicate #3 | 9.9 | -8.9 | -12.3 | -4.7 | -3.5 | -11.5 |
| MDA-MB-231 | 12.1 | -13.1 | -13.3 | -7.8 | -4.7 | -2.1 |
| breast cancer cell line_MDA-MB-231 | 12.1 | -13.1 | -13.3 | -7.9 | -4.7 | -2.1 |
| Breast cancer cell line MDA-MB-231 WT (expression data) | 9.9 | -12.3 | -12.5 | -4.1 | -2.0 | -9.8 |
| MDA-MB-231_control | 12.3 | -12.9 | -11.6 | -2.4 | -1.5 | -0.2 |
| MDA-MB-231_control (2) | 12.8 | -15.6 | -9.2 | -15.5 | -1.6 | -12.2 |
| MDA-MB-231 control ETOH (vehicle) | 11.9 | -15.0 | -8.9 | -9.2 | -1.7 | -8.7 |
| MDA-MB-231T_originating cell line_R1 | 10.6 | -14.6 | -14.6 | 2.9 | -0.5 | -0.6 |
| MDA-MB-231T_originating cell line_R2 | 11.4 | -14.5 | -14.4 | 3.1 | -0.8 | -0.6 |
| Serum starved MDA-MB-231 cells, Rep1 | 5.8 | -13.7 | -15.8 | 2.6 | -2.0 | 2.2 |
| Serum starved MDA-MB-231 cells, Rep2 | 6.8 | -13.4 | -15.0 | 2.8 | -2.0 | 2.4 |
| MDA-MB-231 | 17.3 | -5.4 | -12.6 | -1.3 | -0.2 | -1.0 |
| MDA-MB-231 cells siControl, biological replica A | 11.0 | -15.4 | -13.5 | -11.2 | -2.2 | -13.6 |
| MDA-MB-231 cells siControl, biological replica B | 11.1 | -15.2 | -13.3 | -11.3 | -1.8 | -13.5 |
| MDA-MB-231 cells siControl, biological replica C | 11.2 | -15.6 | -14.5 | -11.6 | -1.7 | -13.0 |
| MDA-MB-231 cells siControl, biological replica D | 10.6 | -15.8 | -14.6 | -11.7 | -1.8 | -13.2 |
| MDA-MB-231 control | 12.3 | -12.9 | -11.7 | -2.4 | -1.5 | -0.2 |
| CellLine, MDA-MB-231 | 11.7 | -12.9 | -12.6 | -2.1 | -0.8 | 2.7 |
| BA CellLine, MDA-MB-231 | 11.7 | -12.9 | -12.6 | -2.1 | -0.8 | 2.7 |
| MDA-MB-231 | 15.0 | -11.4 | -11.1 | -4.8 | -1.0 | -8.6 |
| MDA-MB-231 | 15.0 | -11.4 | -11.1 | -4.8 | -1.0 | -8.6 |

EP 4 039 825 A1

D (continued)

| title | NOTCH log2odds. | STAT1/2-II log2odds. | STAT1/2-S log2odds.S | STAT1/2-I log2odds. | STAT3-E log2odds. | TGFB log2odds. | WNT log2odds. |
|---|---|---|---|---|---|---|---|
| MDA-MB-231, control, replicate #1 | 0.2 | | -9.5 | -8.7 | 6.5 | -1.3 | -10.4 |
| MDA-MB-231, control, replicate #2 | 2.3 | | -9.3 | -8.7 | 8.0 | 0.7 | -11.3 |
| MDA-MB-231, control, replicate #3 | -1.4 | | -8.0 | -6.4 | 5.6 | -1.8 | -8.1 |
| MDA-MB-231 | 3.0 | | -8.5 | -8.4 | 9.7 | -0.3 | -3.9 |
| breast cancer cell line _MDA-MB-231 | 3.0 | | -8.5 | -8.4 | 9.7 | -0.3 | -4.0 |
| Breast cancer cell line MDA-MB-231 WT (expression data) | 3.2 | | -10.0 | -9.9 | 7.7 | -0.8 | -11.3 |
| MDA-MB-231_control | 7.2 | | -9.6 | -10.4 | 7.3 | 3.1 | -3.6 |
| MDA-MB-231_control (2) | 4.1 | | -9.3 | -10.7 | 9.1 | -1.3 | -14.7 |
| MDA-MB-231 control ETOH (vehicle) | 9.0 | | -9.1 | -10.4 | 7.9 | 5.4 | -9.9 |
| MDA-MB-231T_originating cell line_R1 | 9.6 | | -10.3 | -11.0 | 5.1 | 3.1 | -1.2 |
| MDA-MB-231T_originating cell line_R2 | 10.7 | | -10.5 | -11.5 | 6.0 | 4.2 | -1.8 |
| Serum starved MDA-MB-231 cells, Rep1 | 8.6 | | -9.7 | -9.8 | 6.9 | 2.1 | -10.1 |
| Serum starved MDA-MB-231 cells, Rep2 | 9.4 | | -10.1 | -10.4 | 7.2 | 3.1 | -10.1 |
| MDA-MB-231 | 10.6 | | -9.3 | -9.7 | 11.2 | 7.5 | 0.1 |
| MDA-MB-231 cells siControl, biological replica A | 0.5 | | -8.8 | -9.1 | 10.4 | -0.9 | -5.8 |
| MDA-MB-231 cells siControl, biological replica B | 2.3 | | -8.7 | -9.3 | 10.2 | -0.7 | -6.2 |
| MDA-MB-231 cells siControl, biological replica C | 0.5 | | -8.6 | -8.9 | 10.1 | -0.9 | -6.7 |
| MDA-MB-231 cells siControl, biological replica D | 0.3 | | -9.1 | -9.5 | 10.1 | -0.7 | -6.5 |
| MDA-MB-231 control | 7.2 | | -9.6 | -10.4 | 7.3 | 3.1 | -3.6 |
| CellLine, MDA-MB-231 | 8.9 | | -9.6 | -10.5 | 7.6 | -0.1 | -4.5 |
| BA CellLine, MDA-MB-231 | 8.9 | | -9.6 | -10.5 | 7.6 | -0.1 | -4.5 |
| MDA-MB-231 | 2.5 | | -9.7 | -10.0 | 10.5 | 4.5 | -5.7 |
| MDA-MB-231 | 2.5 | | -9.7 | -10.0 | 10.5 | 4.5 | -5.7 |

Fig. 5 (continued)

Fig. 5 (continued)

E

| group | dataset | QC.passed | QC.man.p | AP1 log2odds. | AR log2odds. | ER log2odds. | FOXO log2odds. | HH log2odds. | NFKB log2odds. | NOTCH log2odds. |
|---|---|---|---|---|---|---|---|---|---|---|
| | | 0 | 0 | 0 | 0 | | | | | |
| BT474 breast cancer cell line, DMSO | GSE16179 | TRUE | TRUE | -13.8 | -10.0 | 0.0 | -8.3 | -11.0 | -14.4 | 10.1 |
| BT474 breast cancer cell line, DMSO | GSE16179 | TRUE | TRUE | -13.9 | -9.8 | -0.2 | -8.6 | -11.6 | -14.8 | 10.1 |
| BT474 breast cancer cell line, DMSO | GSE16179 | TRUE | TRUE | -12.8 | -10.2 | -0.9 | -9.2 | -11.4 | -14.5 | 10.8 |
| BT-474_Control siRNA_Vehicle (0.001% Ethanol)_24 hrs | GSE71347 | TRUE | TRUE | -10.4 | -9.7 | -13.6 | -7.7 | -10.2 | -15.8 | 5.9 |
| BT-474_Control siRNA_Vehicle (0.001% Ethanol)_24 hrs | GSE71347 | TRUE | TRUE | -11.1 | -9.9 | -13.6 | -6.6 | -10.6 | -16.2 | 7.1 |
| BT-474_Control siRNA_Vehicle (0.001% Ethanol)_24 hrs | GSE71347 | TRUE | TRUE | -13.0 | -9.4 | -11.6 | -6.0 | -10.2 | -16.7 | 5.5 |
| BT-474_Control siRNA_Vehicle (0.001% Ethanol)_4 hrs | GSE71347 | TRUE | TRUE | -9.1 | -8.9 | -15.9 | -7.0 | -10.3 | -15.6 | 6.4 |
| BT-474_Control siRNA_Vehicle (0.001% Ethanol)_4 hrs | GSE71347 | TRUE | TRUE | -10.8 | -9.8 | -14.7 | -6.2 | -9.9 | -16.1 | 7.9 |
| BT-474_Control siRNA_Vehicle (0.001% Ethanol)_4 hrs | GSE71347 | TRUE | TRUE | -11.9 | -9.6 | -14.7 | -5.5 | -9.4 | -16.5 | 6.9 |
| Wildtype BT474 cells | GSE15043 | TRUE | TRUE | -5.2 | -9.9 | 5.1 | 3.6 | -10.2 | -12.5 | 16.2 |
| Wildtype BT474 cells | GSE15043 | TRUE | TRUE | -6.7 | -10.0 | 4.7 | 1.6 | -10.8 | -12.5 | 16.2 |
| Breast | EMTAB37 | TRUE | | -15.5 | -11.4 | -2.3 | -7.6 | -11.5 | -15.4 | 6.2 |
| Breast | EMTAB37 | TRUE | | -14.1 | -14.2 | -8.0 | -9.5 | -10.7 | -14.0 | 3.9 |
| Breast | EMTAB37 | TRUE | | -15.5 | -13.7 | -7.0 | -8.9 | -10.8 | -14.8 | 5.2 |
| BT474 cell line | GSE10843 | TRUE | | -16.0 | -13.1 | 2.0 | -2.8 | -12.7 | -13.8 | 11.3 |
| Breast cancer | GSE12790 | TRUE | | -16.0 | -13.1 | 2.0 | -2.8 | -12.8 | -13.8 | 11.3 |
| breast | GSE34211 | FALSE | | -6.5 | -8.7 | -9.2 | -0.7 | -13.2 | -12.8 | 9.8 |
| BT474 cell line | GSE50820 | TRUE | TRUE | -15.0 | -10.3 | -9.4 | -0.4 | -8.9 | -13.3 | 8.7 |
| BT474 cell line | GSE50820 | FALSE | TRUE | -14.5 | -10.4 | -12.2 | -4.1 | -9.3 | -14.3 | 8.9 |
| BT474 cell line | GSE50820 | FALSE | TRUE | -13.4 | -10.0 | -21.0 | -1.3 | -7.5 | -13.9 | 3.2 |
| BT474 cell line | GSE50820 | FALSE | TRUE | -12.9 | -5.9 | -18.5 | -4.4 | -6.8 | -13.6 | 5.4 |
| ATCC | GSE87006 | TRUE | | -13.5 | -9.9 | -5.0 | 0.7 | -12.4 | -14.7 | 8.4 |
| ATCC | GSE87008 | TRUE | TRUE | -13.5 | -9.9 | -5.0 | 0.7 | -12.4 | -14.7 | 8.4 |

E (continued)

| group | STAT1/2-II log2odds.S | STAT1/2-I log2odds. | STAT3-E log2odds. | TGFB log2odds. | WNT log2odds. |
|---|---|---|---|---|---|
| BT474 breast cancer cell line, DMSO | -7.5 | -10.1 | 4.3 | -14.4 | -8.6 |
| BT474 breast cancer cell line, DMSO | -7.4 | -9.8 | 4.2 | -15.1 | -7.6 |
| BT474 breast cancer cell line, DMSO | -7.2 | -9.6 | 2.9 | -16.2 | -9.0 |
| BT-474_Control siRNA_Vehicle (0.001% Ethanol)_24 hrs | -6.3 | -8.4 | 0.7 | -13.0 | -4.6 |
| BT-474_Control siRNA_Vehicle (0.001% Ethanol)_24 hrs | -6.4 | -8.5 | 0.3 | -13.9 | -4.7 |
| BT-474_Control siRNA_Vehicle (0.001% Ethanol)_24 hrs | -6.9 | -8.9 | -0.1 | -15.8 | -5.1 |
| BT-474_Control siRNA_Vehicle (0.001% Ethanol)_4 hrs | -7.1 | -8.8 | 1.5 | -11.0 | -3.4 |
| BT-474_Control siRNA_Vehicle (0.001% Ethanol)_4 hrs | -7.1 | -9.0 | 0.6 | -12.6 | -3.3 |
| BT-474_Control siRNA_Vehicle (0.001% Ethanol)_4 hrs | -6.8 | -8.7 | 1.2 | -14.4 | -3.9 |
| Wildtype BT474 cells | -7.7 | -9.1 | 3.7 | -12.9 | -3.4 |
| Wildtype BT474 cells | -7.5 | -9.3 | 3.9 | -13.3 | -3.7 |
| Breast | -6.7 | -9.4 | 1.5 | -18.4 | -9.8 |
| Breast | -5.6 | -7.6 | -1.2 | -19.3 | -10.2 |
| Breast | -5.7 | -8.0 | -1.1 | -18.8 | -9.7 |
| BT474 cell line | -7.7 | -10.1 | 2.7 | -17.9 | -7.0 |
| Breast cancer | -7.7 | -10.1 | 2.6 | -17.9 | -7.0 |
| breast | -8.4 | -9.3 | 5.3 | -14.0 | -0.5 |
| BT474 cell line | -8.0 | -9.8 | -1.5 | -20.3 | -3.0 |
| BT474 cell line | -8.3 | -10.0 | -0.9 | -20.7 | -5.1 |
| BT474 cell line | -8.2 | -9.7 | -3.1 | -18.3 | -6.8 |
| BT474 cell line | -8.3 | -10.2 | -0.9 | -18.6 | -3.1 |
| ATCC | -7.4 | -8.9 | 0.2 | -16.6 | -8.9 |
| ATCC | -7.4 | -8.9 | 0.2 | -16.6 | -8.9 |

Fig. 5 (continued)

| array | title | Passage | dataset | QC.passed | AP1 | AE | ER | FOXO | HH | NFKB |
|---|---|---|---|---|---|---|---|---|---|---|
| GSM1182325 | Ts21 iPSC DS1 biological replicate 1 | 24 | GSE48611 | TRUE | -4.1 | -8.1 | -9.8 | -7.7 | 5.0 | -9.8 |
| GSM1182326 | Ts21 iPSC DS1 biological replicate 2 | 25 | GSE48611 | TRUE | -4.2 | -7.7 | -9.7 | -7.6 | 5.3 | -8.4 |
| GSM1182327 | Ts21 iPSC DS1 biological replicate 3 | 31 | GSE48611 | TRUE | -3.7 | -6.9 | -9.6 | -6.6 | 4.4 | -8.4 |
| GSM1182328 | Ts21 iPSC DS4 biological replicate 1 | 28 | GSE48611 | TRUE | -3.5 | -7.0 | -9.7 | -7.1 | 6.2 | -6.6 |
| GSM1182329 | Ts21 iPSC DS4 biological replicate 2 | 29 | GSE48611 | TRUE | -2.6 | -7.8 | -9.8 | -7.2 | 5.3 | -5.5 |
| GSM1182330 | Ts21 iPSC DS4 biological replicate 3 | 34 | GSE48611 | TRUE | -3.7 | -7.8 | -9.8 | -7.2 | 5.8 | -8.4 |
| GSM1182331 | Euploid iPSC DS2U biological replicate 1 | 46 | GSE48611 | TRUE | -0.7 | -7.8 | -10.3 | -5.8 | 3.8 | -4.7 |
| GSM1182332 | Euploid iPSC DS2U biological replicate 2 | 47 | GSE48611 | TRUE | -0.3 | -7.6 | -10.1 | -7.5 | 5.3 | -4.7 |
| GSM1182333 | Euploid iPSC DS2U biological replicate 3 | 48 | GSE48611 | TRUE | -0.6 | -7.5 | -10.0 | -7.7 | 7.1 | -2.6 |

F

Fig. 5 (continued)

Fig. 5 (continued)

EP 4 039 825 A1

F (continued)

| title | NOTCH | STAT1-2 INFII | STAT1-2 INFI | STAT3 | STAT3-B | TGFB | WNT |
|---|---|---|---|---|---|---|---|
| Ts21 iPSC DS1 biological replicate 1 | -2.6 | -10.0 | -10.0 | -8.1 | 2.5 | -4.3 | -15.2 |
| Ts21 iPSC DS1 biological replicate 2 | -2.2 | -10.1 | -10.4 | -8.4 | 3.1 | -3.8 | -15.2 |
| Ts21 iPSC DS1 biological replicate 3 | -2.0 | -10.1 | -10.2 | -7.4 | 3.4 | -2.9 | -14.1 |
| Ts21 iPSC DS4 biological replicate 1 | -1.3 | -10.3 | -10.5 | -8.5 | 3.3 | -4.2 | -13.8 |
| Ts21 iPSC DS4 biological replicate 2 | -2.2 | -10.2 | -10.1 | -8.8 | 1.1 | -4.3 | -15.7 |
| Ts21 iPSC DS4 biological replicate 3 | -2.1 | -9.9 | -10.3 | -7.3 | 1.4 | -4.0 | -15.3 |
| Euploid iPSC DS2U biological replicate 1 | -1.8 | -10.1 | -9.8 | -7.5 | 2.9 | -4.1 | -14.4 |
| Euploid iPSC DS2U biological replicate 2 | -2.0 | -9.9 | -10.0 | -7.8 | 1.4 | -3.5 | -14.3 |
| Euploid iPSC DS2U biological replicate 3 | -3.0 | -9.7 | -10.2 | -9.7 | 0.4 | -3.4 | -14.3 |

Fig. 6

| Cell line | Pass. Nr. | AR | | ER | | FOXO | | HH | | TGFbeta | | WNT | | PI3K | |
|---|---|---|---|---|---|---|---|---|---|---|---|---|---|---|---|
| | | AV | SD | AV | SD | AV | SD | AV | SD | AV | SD | AV | SD | AV | SD |
| T47D p6 | 6 | 14.49 | 0.16 | 52.16 | 0.72 | 35.78 | 0.41 | 21.02 | 1.59 | 13.30 | 0.23 | 17.90 | 0.02 | 64.22 | 0.41 |
| T47D p12 | 12 | 11.30 | 0.37 | 48.77 | 1.51 | 34.59 | 0.17 | 21.47 | 2.13 | 21.87 | 0.35 | 17.83 | 0.06 | 65.41 | 0.17 |
| T47D p20 | 20 | 11.38 | 0.36 | 51.81 | 0.86 | 35.54 | 0.22 | 19.61 | 0.29 | 15.59 | 0.55 | 17.20 | 0.54 | 64.46 | 0.22 |
| MCF7 p6 | 6 | 17.40 | 0.38 | 52.95 | 0.44 | 37.29 | 1.12 | 17.13 | 0.09 | 37.55 | 1.82 | 9.38 | 0.28 | 62.71 | 1.12 |
| MCF7 p13 | 13 | 13.37 | 0.36 | 51.43 | 2.07 | 14.43 | 1.37 | 15.54 | 0.16 | 21.46 | 0.80 | 7.41 | 0.18 | 85.57 | 1.37 |
| MCF7 p21 | 21 | 12.97 | 0.14 | 72.70 | 0.67 | 29.62 | 1.30 | 15.63 | 0.00 | 30.10 | 0.91 | 7.92 | 0.08 | 70.38 | 1.30 |

Fig. 7

A

| Pre Day 0 | Regular set up and culture CL's: |
|---|---|
| | 1 vial U87MG set up with culture medium, incl. 10%hiFBS |
| | 1 vial U87MG set up with culture medium, incl. 10%nonFBS |
| | 1 vial MO59K set up with culture medium, incl. 10%hiFBS |
| | --> cultured for 4 passages |
| Day 0 | Set up 3xT25 flasks from each regular culture: |
| | 1xT25 with *regular* medium (=incl. **10% hi FBS** ) |
| | 1xT25 with medium (=incl. **10% nonFBS** ) |
| | 1xT25 with medium (=incl. **0, 5% hiFBS** ) |
| | Culture for 7 days without refreshing and disturbing |

Fig. 7 (continued)

B

| Group: | AR | ER | FOXO | HH | TGFbeta | WNT | PI3K |
|---|---|---|---|---|---|---|---|
| M059K_10%hiFBS + 10%hiFBS | 20.8 | 9.8 | 55.9 | 25.4 | 64.3 | 30.4 | 44.1 |
| M059K_10%hiFBS + 10% noniFBS | 20.9 | 5.6 | 51.5 | 21.6 | 67.9 | 30.8 | 48.5 |
| M059K_10%hiFBS + 0,5%hiFBS | 22.9 | 5.4 | 44.5 | 22.4 | 52.8 | 24.5 | 55.5 |
| U87MG_10%hiFBS + 10%hiFBS | 20.7 | 22.9 | 62.9 | 28.6 | 64.7 | 27.0 | 37.1 |
| U87MG_10%hiFBS + 10% noniFBS | 20.7 | 22.6 | 63.1 | 29.6 | 60.6 | 23.1 | 36.9 |
| U87MG_10%hiFBS + 0,5%hiFBS | 20.7 | 25.3 | 66.1 | 30.4 | 65.9 | 32.6 | 33.9 |
| U87MG_10%noniFBS + 10%hiFBS | 20.7 | 23.8 | 63.4 | 29.0 | 65.5 | 30.2 | 36.6 |
| U87MG_10%noniFBS + 10% noniFBS | 20.7 | 22.9 | 62.7 | 29.5 | 64.3 | 27.0 | 37.3 |
| U87MG_10%noniFBS + 0,5%hiFBS | 20.7 | 23.9 | 61.7 | 31.0 | 63.8 | 30.6 | 38.3 |

Fig. 8

| array | group | MAPK-AP1 | AR | ER | FOXO | HH | NOTCH | TGFb |
|---|---|---|---|---|---|---|---|---|
| - | Ls174T | 13,1 | 2,4 | 13,8 | 24,5 | 21,2 | 58,8 | 16,6 |
| - | Ls174T | 12,5 | 1,9 | 12,2 | 23,8 | 21,1 | 58,1 | 15,8 |
| - | Ls174T | 13,9 | 2,4 | 12,9 | 24,2 | 21,0 | 59,7 | 15,8 |
| GSM461946 | Ls174T, <U+03B2>-catenin siRNA, induced | 26,9 | 5,2 | 7,0 | 31,3 | 21,7 | 57,4 | 11,1 |
| GSM461948 | Ls174T, <U+03B2>-catenin siRNA, induced | 27,6 | 3,7 | 5,9 | 32,7 | 21,8 | 58,0 | 13,5 |
| GSM461950 | Ls174T, <U+03B2>-catenin siRNA, induced | 30,5 | 3,7 | 6,7 | 29,7 | 23,1 | 58,1 | 12,5 |
| GSM461942 | Ls174T, <U+03B2>-catenin siRNA, uninduced | 21,1 | 4,6 | 5,8 | 24,8 | 23,8 | 63,2 | 14,5 |
| GSM461947 | Ls174T, <U+03B2>-catenin siRNA, uninduced | 23,1 | 5,8 | 6,1 | 25,6 | 24,3 | 63,0 | 15,0 |
| GSM461949 | Ls174T, <U+03B2>-catenin siRNA, uninduced | 20,5 | 5,0 | 7,2 | 26,7 | 23,2 | 64,4 | 15,5 |
| GSM461943 | Ls174T, dominant-negative Tcf4, induced | 36,0 | 7,5 | 10,0 | 31,0 | 19,1 | 51,1 | 15,7 |
| GSM461945 | Ls174T, dominant-negative Tcf4, induced | 38,9 | 6,0 | 13,2 | 30,5 | 19,0 | 52,6 | 16,5 |
| GSM461952 | Ls174T, dominant-negative Tcf4, induced | 39,4 | 7,1 | 10,5 | 30,2 | 18,8 | 49,4 | 14,6 |
| GSM461944 | Ls174T, dominant-negative Tcf4, uninduced | 37,5 | 5,7 | 7,4 | 22,6 | 19,9 | 51,0 | 10,3 |
| GSM461951 | Ls174T, dominant-negative Tcf4, uninduced | 42,1 | 6,1 | 7,5 | 24,4 | 20,7 | 52,2 | 11,0 |
| GSM461953 | Ls174T, dominant-negative Tcf4, uninduced | 37,4 | 6,0 | 6,2 | 23,8 | 20,3 | 51,2 | 11,1 |

Fig. 8 (continued)

| array | group | NFkB | STAT1/2 | STAT3 | WNT |
|---|---|---|---|---|---|
| - | Ls174T | 20,7 | 12,4 | 45,1 | 78,6 |
| - | Ls174T | 20,2 | 12,1 | 43,7 | 78,6 |
| - | Ls174T | 20,0 | 13,1 | 45,4 | 78,2 |
| GSM461946 | Ls174T, <U+03B2>-catenin siRNA, induced | 26,9 | 32,4 | 50,2 | 55,3 |
| GSM461948 | Ls174T, <U+03B2>-catenin siRNA, induced | 24,8 | 38,4 | 50,6 | 56,8 |
| GSM461950 | Ls174T, <U+03B2>-catenin siRNA, induced | 27,7 | 35,6 | 51,4 | 55,6 |
| GSM461942 | Ls174T, <U+03B2>-catenin siRNA, uninduced | 24,5 | 30,8 | 64,3 | 80,1 |
| GSM461947 | Ls174T, <U+03B2>-catenin siRNA, uninduced | 24,7 | 31,5 | 65,1 | 79,3 |
| GSM461949 | Ls174T, <U+03B2>-catenin siRNA, uninduced | 25,2 | 29,4 | 65,8 | 79,6 |
| GSM461943 | Ls174T, dominant-negative Tcf4, induced | 28,5 | 39,2 | 65,6 | 47,7 |
| GSM461945 | Ls174T, dominant-negative Tcf4, induced | 29,3 | 38,0 | 66,7 | 42,9 |
| GSM461952 | Ls174T, dominant-negative Tcf4, induced | 28,4 | 36,6 | 67,3 | 47,5 |
| GSM461944 | Ls174T, dominant-negative Tcf4, uninduced | 26,8 | 22,3 | 71,0 | 83,6 |
| GSM461951 | Ls174T, dominant-negative Tcf4, uninduced | 27,7 | 24,2 | 71,1 | 83,3 |
| GSM461953 | Ls174T, dominant-negative Tcf4, uninduced | 27,2 | 22,5 | 70,3 | 80,7 |

A

High confluency

Low confluency

Fig. 9

Fig. 9 (continued)

C

| Group | AP1 | | AR | | ER | | FOXO | |
|---|---|---|---|---|---|---|---|---|
| | Average | STDEV | Average | STDEV | Average | STDEV | Average | STDEV |
| ovcar3_High Density | 28.4 | 2.5 | 14.7 | 0.7 | 8.8 | 1.1 | 58.0 | 2.1 |
| ovcar3_Low Density | 32.8 | 0.5 | 13.8 | 1.2 | 5.6 | 0.7 | 47.6 | 3.0 |

| Group | HH | | NOTCH | | TGFB | | PI3K | |
|---|---|---|---|---|---|---|---|---|
| | Average | STDEV | Average | STDEV | Average | STDEV | Average | STDEV |
| ovcar3_High Density | 28.2 | 0.7 | 29.5 | 4.4 | 14.5 | 2.1 | 42.0 | 2.1 |
| ovcar3_Low Density | 29.1 | 0.1 | 36.8 | 2.7 | 17.1 | 0.4 | 52.4 | 3.0 |

Fig. 9 (continued)

Europäisches
Patentamt

European
Patent Office

Office européen
des brevets

## EUROPEAN SEARCH REPORT

Application Number

EP 21 15 5943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2019/068623 A1 (KONINKLIJKE PHILIPS NV [NL]) 11 April 2019 (2019-04-11) | 1-10 | INV. C12Q1/6881 C12Q1/6883 C12Q1/6886 |
| Y | * claims 1-15 * <br> * page 7, line 26 - page 8, line 17 * <br> * page 16, line 23 - page 19, line 7 * <br> * page 28 - page 31 * <br> ----- | 7,10 | |
| X,D | EP 3 461 916 A1 (KONINKLIJKE PHILIPS NV [NL]) 3 April 2019 (2019-04-03) <br> * claims 1-13 * <br> * paragraphs [0011], [0024] - [0026], [0051] - [0052], [0058], [0074] * <br> * figure 10 * <br> * table 1 * <br> ----- | 1-10 | |
| X | PARIKH JIGNESH R. ET AL: "Discovering causal signaling pathways through gene-expression patterns", NUCLEIC ACIDS RESEARCH, vol. 38, no. suppl_2, 1 July 2010 (2010-07-01), pages W109-W117, XP055819363, GB ISSN: 0305-1048, DOI: 10.1093/nar/gkq424 Retrieved from the Internet: URL:https://www.ncbi.nlm.nih.gov/pmc/artic les/PMC2896193/pdf/gkq424.pdf> * the whole document * <br> -/-- | 1-10 | |

TECHNICAL FIELDS
SEARCHED        (IPC)

C12Q

The present search report has been drawn up for all claims

| Place of search | Date of completion of the search | Examiner |
|---|---|---|
| The Hague | 30 June 2021 | Bruma, Anja |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 21 15 5943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | & Parikh Jignesh R ET AL: "Supplementary Data of Discovering causal signaling pathways through gene-expression patterns", Nucleic Acids Research, 21 May 2010 (2010-05-21), XP055819674, Retrieved from the Internet: URL:https://www.biorxiv.org/content/10.1101/2020.05.13.092759v1.full.pdf [retrieved on 2021-06-30] * the whole document * | 1-10 | |
| X | Bouwman Wilbert ET AL: "Quantitative measurement of activity of JAK-STAT signaling pathways in blood samples and immune cells to predict innate and adaptive cellular immune response to viral infection and accelerate vaccine development.", , 15 May 2020 (2020-05-15), pages 1-28, XP055786433, DOI: 10.1101/2020.05.13.092759 Retrieved from the Internet: URL:https://www.biorxiv.org/content/10.1101/2020.05.13.092759v1.full.pdf [retrieved on 2021-03-16] * the whole document * -/-- | 1-4,6-10 | TECHNICAL FIELDS SEARCHED (IPC) |

The present search report has been drawn up for all claims

| Place of search | Date of completion of the search | Examiner |
|---|---|---|
| The Hague | 30 June 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 15 5943

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | & Bouwman Wilbert ET AL: "Quantitative measurement of activity of JAK-STAT signaling pathways in blood samples and immune cells to predict innate and adaptive cellular immune response to viral infection and accelerate vaccine development - suppl. information", , 15 May 2020 (2020-05-15), pages 1-12, XP055786440, DOI: 10.1101/2020.05.13.092759 Retrieved from the Internet: URL:https://www.biorxiv.org/content/10.1101/2020.05.13.092759v1.supplementary-material [retrieved on 2021-03-16] * the whole document * | 1-4,6-10 | |
| X | US 2012/252689 A1 (WU ZHONG [US] ET AL) 4 October 2012 (2012-10-04) * claims 1-27 * * paragraphs [0046] - [0050] * * example 1 * | 1-3,6-10 | |
| Y | WO 2006/124836 A1 (UNIV DUKE [US]; NEVINS JOSEPH R [US] ET AL.) 23 November 2006 (2006-11-23) * claims 1-28 * * example 2 * * table suppl. 1 * | 7,10 | TECHNICAL FIELDS SEARCHED (IPC) |

The present search report has been drawn up for all claims

| Place of search | Date of completion of the search | Examiner |
|---|---|---|
| The Hague | 30 June 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 21 15 5943

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2021

| Patent document cited in search report | | | Publication date | Patent family member(s) | | | Publication date |
|---|---|---|---|---|---|---|---|
| WO 2019068623 | A1 | | 11-04-2019 | BR | 112020006605 | A2 | 06-10-2020 |
| | | | | CA | 3077501 | A1 | 11-04-2019 |
| | | | | CN | 111587293 | A | 25-08-2020 |
| | | | | EP | 3692170 | A1 | 12-08-2020 |
| | | | | JP | 2020535847 | A | 10-12-2020 |
| | | | | US | 2020240979 | A1 | 30-07-2020 |
| | | | | WO | 2019068623 | A1 | 11-04-2019 |
| EP 3461916 | A1 | | 03-04-2019 | CN | 111448325 | A | 24-07-2020 |
| | | | | EP | 3461916 | A1 | 03-04-2019 |
| | | | | EP | 3692172 | A1 | 12-08-2020 |
| | | | | JP | 2020535823 | A | 10-12-2020 |
| | | | | US | 2019102510 | A1 | 04-04-2019 |
| | | | | WO | 2019068543 | A1 | 11-04-2019 |
| US 2012252689 | A1 | | 04-10-2012 | EP | 2694963 | A1 | 12-02-2014 |
| | | | | JP | 6355555 | B2 | 11-07-2018 |
| | | | | JP | 2014512177 | A | 22-05-2014 |
| | | | | US | 2012252689 | A1 | 04-10-2012 |
| | | | | WO | 2012135845 | A1 | 04-10-2012 |
| WO 2006124836 | A1 | | 23-11-2006 | CA | 2608359 | A1 | 23-11-2006 |
| | | | | EP | 1910564 | A1 | 16-04-2008 |
| | | | | US | 2009186024 | A1 | 23-07-2009 |
| | | | | WO | 2006124836 | A1 | 23-11-2006 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013011479 A
- WO 2014102668 A
- WO 2015101635 A
- WO 2016062891 A
- WO 2017029215 A
- WO 2014174003 A
- WO 2016062892 A
- WO 2016062893 A
- WO 2018096076 A
- WO 2019068585 A
- WO 2019120658 A
- WO 2019068543 A
- WO 2019068562 A
- WO 2019068623 A
- WO 2013011479 A2
- WO 2014102668 A2

**Non-patent literature cited in the description**

- **M.R. GREEN ; J. SAMBROOK.** Molecular Cloning: A Laboratory Manual. Cold Spring Harbor Laboratory Press
- **W VERHAEGH et al.** Selection of personalized patient therapy through the use of knowledge-based computational models that identify tumor-driving signal transduction pathways. *Cancer Research,* 2014, vol. 74 (11), 2936-2945
- **C. A. CHACÓN-MARTÍNEZ ; J. KOESTER ; S. A. WICKSTRÖM.** Signaling in the stem cell niche: regulating cell fate, function and plasticity. *Development,* August 2018, vol. 145 (15), 165399
- **L.C. CANTLEY ; T. HUNTER ; R. SEVER ; J. W. THORNER.** Signal transduction: principles, pathways, and processes. Cold Spring Harbor Laboratory Press, 2014
- **C. MUMMERY ; A. VAN DE STOLPE ; B. ROELEN ; H. CLEVERS.** Stem Cells: Scientific Facts and Fiction. Academic Press, 2014
- **J. S. L. YU ; W. CUI.** Proliferation, survival and metabolism: the role of PI3K/AKT/mTOR signalling in pluripotency and cell fate determination. *Development,* September 2016, vol. 143 (17), 3050-3060
- **J.-F. ARNAL et al.** Membrane and Nuclear Estrogen Receptor Alpha Actions: From Tissue Specificity to Medical Implications. *Physiol. Rev.,* 2017, vol. 97 (3), 1045-1087
- **M. KONO ; T. FUJII ; B. LIM ; M. S. KARUTURI ; D. TRIPATHY ; N. T. UENO.** Androgen Receptor Function and Androgen Receptor-Targeted Therapies in Breast Cancer: A Review. *JAMA Oncol,* September 2017, vol. 3 (9), 1266-1273
- **C. SIEBEL ; U. LENDAHL.** Notch Signaling in Development, Tissue Homeostasis, and Disease. *Physiol. Rev.,* 2017, vol. 97 (4), 1235-1294
- **M. BUROTTO ; V. L. CHIOU ; J.-M. LEE ; E. C. KOHN.** The MAPK pathway across different malignancies: a new perspective. *Cancer,* November 2014, vol. 120 (22), 3446-3456
- **E. PARDALI ; G. SANCHEZ-DUFFHUES ; M. C. GOMEZ-PUERTO ; P. TEN DIJKE.** TGF-β-Induced Endothelial-Mesenchymal Transition in Fibrotic Diseases. *Int J Mol Sci,* October 2017, vol. 18 (10
- **G. SÁNCHEZ-DUFFHUES ; A. GARCIA DE VINUESA ; P. TEN DIJKE.** Endothelial-to-mesenchymal transition in cardiovascular diseases: Developmental signaling pathways gone awry. *Dev. Dyn.,* 2018, vol. 247 (3), 492-508
- **F. A. HIGH ; J. A. EPSTEIN.** The multifaceted role of Notch in cardiac development and disease. *Nat. Rev. Genet.,* January 2008, vol. 9 (1), 49-61
- **G. SANCHEZ-DUFFHUES ; V. ORLOVA ; P. TEN DIJKE.** In Brief: Endothelial-to-mesenchymal transition. *J. Pathol.,* February 2016, vol. 238 (3), 378-380
- **M. BIENZ ; H. CLEVERS.** Linking colorectal cancer to Wnt signaling. *Cell,* October 2000, vol. 103 (2), 311-320
- **S. S. KARHADKAR et al.** Hedgehog signalling in prostate regeneration, neoplasia and metastasis. *Nature,* October 2004, vol. 431 (7009), 707-712
- **J. TAIPALE ; P. A. BEACHY.** The Hedgehog and Wnt signalling pathways in cancer. *Nature,* May 2001, vol. 411 (6835), 349-354
- **D. HANAHAN ; R. A. WEINBERG.** Hallmarks of cancer: the next generation. *Cell,* March 2011, vol. 144 (5), 646-674
- **A. VAN DE STOLPE.** On the origin and destination of cancer stem cells: a conceptual evaluation. *Am J Cancer Res,* 2013, vol. 3 (1), 107-116

- **T. A. BAUDINO.** Targeted Cancer Therapy: The Next Generation of Cancer Treatment. *Curr Drug Discov Technol,* 2015, vol. 12 (1), 3-20
- **Y. K. CHAE et al.** Path toward Precision Oncology: Review of Targeted Therapy Studies and Tools to Aid in Defining 'Actionability' of a Molecular Lesion and Patient Management Support. *Mol Cancer Ther,* December 2017, vol. 16 (12), 2645-2655
- **U. BEN-DAVID et al.** Genetic and transcriptional evolution alters cancer cell line drug response. *Nature,* 2018, vol. 560 (7718), 325-330